# EUROPEAN PATENT APPLICATION

(11) **EP 4 509 521 A1**
(43) Date of publication of application: **19.02.2025**
(21) Application number: 23787575.2
(22) Date of filing: 06.04.2023
(51) Int. Cl.: C07K 14/47, C12N 15/62, A61K 38/17

(54) **COMPLEMENT-INHIBITING HYBRID PROTEIN**

(30) Priority: 11.04.2022 CN 202210374811
(71) Applicant: Longbio Pharma (Suzhou) Co., Ltd., Shanghai 201203 (CN)
(72) Inventor: MA, Haili, Shanghai 201203 (CN); LIU, Heng, Shanghai 201203 (CN)
(74) Representative: Sagittarius IP
(86) International application number: PCT/CN2023/086593
(87) International publication number: WO 2023/197930

(57) **Abstract**

Provided are a hybrid protein comprising a CFH functional unit and a DAF functional unit, an encoding nucleic acid thereof, and a method and use thereof for treating related diseases or disorders of a complement system.

## Description

The present invention relates to a hybrid protein comprising a CFH functional unit and a DAF functional unit, an encoding nucleic acid thereof, and a method and a use thereof for treating a disease or a disorder related to the complement system.

### Background

As part of the innate immune system, the complement system comprises more than 30 kinds of soluble protein molecules and more than 30 kinds of molecules such as intrinsic complement components, various regulatory factors, and complement receptors. It is well known that the complement system acts directly on pathogens to promote pathogen-specific adaptive immune responses; and participates in cell differentiation and polarization, tissue regeneration, lipid metabolism, clearance of immune complexes, and apoptosis, etc. As the deficient or inappropriate complement activation regulation will lead to damage to host tissues, the complement system is tightly regulated by a series of proteins, wherein complement activation regulator (RCA) family proteins are primarily responsible for complement regulation; RCA proteins include membrane proteins such as decay-accelerating factor (DAF; CD55), membrane cofactor protein (MCP; CD46) and complement receptor 1 (CR1; CD35), as well as fluid-phase proteins such as Factor H (FH or CFH) and C4b-binding protein (C4BP). The RCA protein structure consists of complement control protein repeat (CCP) modules, of which 2-4 consecutive modules contribute to regulatory functions known as decay-accelerating activity (DAA) and cofactor activity (CFA). RCA proteins act by targeting the C3/C5 convertase, the central enzyme of the complement pathway. RCA proteins bind to these convertases or their non-catalytic subunits to inactivate these enzymes. The DAA was displayed as the irreversible dissociation of the invertase into subunits after binding to the RCA protein, whereas the CFA was displayed as the cleavage and inactivation of the invertase via the recruitment of serine protease Factor I (FI) after the binding RCA protein to the non-catalytic subunits (C3b/C4b), thereby preventing the formation of C3 convertase. Factor H is an opsonin and ligand for complement receptors 2 and 3, acting as a cofactor for Factor I in the catalyzed cleavage of C3b to iC3b, and inhibiting C3b amplification. It accelerates the irreversible dissociation of C3 convertase C3bBb in the alternative pathway, and may also compete with Factor B for C3B binding during the formation of proconvertase. Factor H is a soluble complement regulator essential for protecting surfaces, including extracellular matrix ECM. FH binds to the peptide hormone adrenomedullin and may prevent its degradation. The FH plays a role in managing cellular senescence, stress, or injury through interactions with C-reactive proteins, pentameric proteins, DNA, histones, annexin II, the malondialdehyde acetaldehyde adduct of proteins, and oxidized lipids. FHL1 also has cofactor activity for Factor I and C3bBb decay-accelerating activity (The Complement FactsBook. Edited by: Scott Barnum and Theresa Schein, Copyright© 2018 Elsevier Ltd. All rights reserved. https://doi.org/10.1016/C2015-0-06595-9, Chapter 30).

DAF intrinsically protects host cells from autologous complement attack by preventing the formation and accelerating the decay of classical and alternative C3 and C5 convertases, thereby inhibiting the cleavage of C3 and C5. When purified DAF is added to cells, it can be incorporated into the cell membrane, thus displaying functional activity. DAF has also been found to regulate T cell tolerance and thus negatively regulate several animal models of autoimmune diseases (The Complement FactsBook. Edited by: Scott Barnum and Theresa Schein, Copyright 2018 Elsevier Ltd. All rights reserved. https://doi.org/10.1016/C2015-0-06595-9, Chapter 25).

The present invention relates to a hybrid protein with better complement regulation activity, and it is hoped that this hybrid protein can have advantages such as better therapeutical effect.

### Description of the Invention

The invention relates to a hybrid protein of CFH and DAF, wherein CCP1/SCR1 of CFH is hybridized with CCP of DAF (e.g., CCP3-4/SCR3-4 of DAF) to form the hybrid protein.

The hybrid proteins have better complement-inhibiting activity, including the activity in inhibiting the Alternative Pathway (AP), the Classical Pathway (CP) and/or C3b deposition.

In some embodiments, the hybrid protein is effective to block human complement hemolytic activity.

In some embodiments, the hybrid protein of the present invention is effective in inhibiting hemolysis of the human complement classical pathway and/or hemolysis of the human complement alternative pathway.

In some embodiments, the hybrid protein of the present invention is effective in inhibiting C3b deposition.

For example, the hybrid protein of the present invention further comprises less CCP of DAF on the basis of comprising CPP1/SCR1 of CFH, so that the hybrid protein having a smaller molecular weight can be obtained. The hybrid proteins, when in the therapeutic applications, can be administered at the same mass concentration with higher molarity doses, and thus have the potential of better therapeutic efficacy or longer dosing intervals, as compared to other typically used complement regulatory proteins of larger molecular weight (especially compared to hybrid proteins that also comprise other CCPs of CFH), e.g. in ophthalmic, brain medications. In other cases, smaller molecular weights are also more conducive to tissue penetration, such as tumor tissue, blood-brain barrier, and the like.

In some embodiments, the present invention relates to the following specific items:
1. A hybrid protein, which comprises or consists of
   (i) CCP1 of human complement Factor H (CFH),
   (ii) CCP3 and CCP4 of human decay-accelerating factors (DAFs), and
   optionally a signal peptide and/or a tag.
2. The hybrid protein of embodiment 1, wherein the CCP3 and CCP4 of DAF are directly linked together to form CCP3-4.
3. The hybrid protein of embodiment 1 or 2, wherein the CCP1 of human CFH comprises or consists of the amino acid sequence at positions 19-82 of the human CFH protein, optionally, the CCP1 comprises a V62I mutation, wherein the amino acid positions are numbered corresponding to the amino acid positions as set forth in SEQ ID NO: 3.
4. The hybrid protein of embodiment 1 or 2, wherein the CCP1 of human CFH comprises or consists of the amino acid sequence at positions 19-84 of the human CFH protein, optionally, the CCP1 comprises a V62I mutation, wherein the amino acid positions are numbered corresponding to the amino acid positions as set forth in SEQ ID NO: 3.
5. The hybrid protein of any one of embodiments 1-4, wherein the CCP3 of human DAF comprises or consists of the amino acid sequence at positions 161-222 of the human DAF protein, and/or the CCP4 of human DAF comprises or consists of the amino acid sequence at positions 223-285 of the human DAF protein, wherein the amino acid positions are numbered corresponding to the amino acid positions as set forth in SEQ ID NO: 1.
6. The hybrid protein of any one of embodiments 1-4, wherein the CCP3 of human DAF comprises or consists of the amino acid sequence 163-222 of the human DAF protein, and/or the CCP4 of human DAF comprises or consists of the amino acid sequence 223-285 of the human DAF protein, wherein the amino acid positions are numbered corresponding to the amino acid positions as set forth in SEQ ID NO: 1.
7. The hybrid protein of embodiment 2, wherein the CCP3-4 of human DAF comprises or consists of the amino acid sequence at positions 161-285 of the human DAF protein, wherein the amino acid positions are numbered corresponding to the amino acid positions as set forth in SEQ ID NO: 1.
8. The hybrid protein of any one of embodiments 2-4, wherein the CCP3-4 of human DAF comprises or consists of the amino acid sequence at position 163-285 of the human DAF protein, wherein the amino acid positions are numbered corresponding to the amino acid positions as set forth in SEQ ID NO: 1.
9. The hybrid protein of embodiment 2, wherein
   (1) the CCP1 of human CFH comprises or consists of the amino acid sequences at positions 19-82 of the human CFH protein, and the CCP3 of human DAF comprises or consists of the amino acid sequence 161-222 of the human DAF protein, and the CCP4 of human DAF comprises or consists of the amino acid sequence 223-285 of the human DAF protein;
   (2) the CCP1 of human CFH comprises or consists of the amino acid sequence at positions 19-84 of the human CFH protein, and the CCP3 of human DAF comprises or consists of the amino acid sequence 163-222 of the human DAF protein, and the CCP4 of human DAF comprises or consists of the amino acid sequence 223-285 of the human DAF protein;
   (3) the CCP1 of human CFH comprises or consists of the amino acid sequence at positions 19-82 of the human CFH protein, and the CCP3-4 of human DAF comprises or consists of the amino acid sequence at positions 161-285 of the human DAF protein; or
   (4) the CCP1 of human CFH comprises or consists of the amino acid sequence at positions 19-84 of the human CFH protein, and the CCP3-4 of human DAF comprises or consists of the amino acid sequence at positions 163-285 of the human DAF protein;
   wherein the amino acid positions of the human CFH protein are numbered corresponding to the amino acid positions as set forth in SEQ ID NO: 3 and the amino acid positions of the human DAF protein are numbered corresponding to the amino acid positions as set forth in SEQ ID NO: 1.
10. The hybrid protein of embodiment 9, wherein the CCP1 of human CFH has a V62I mutation.
11. The hybrid protein of any one of embodiments 1-10, wherein the human CFH protein is a native human CFH protein or comprises
   (i) the amino acid sequence as set forth in SEQ ID NO: 3 or 5;
   (ii) the amino acid sequence encoded by the nucleotide sequence as set forth in SEQ ID NO: 4 or 6;
   (iii) the amino acid sequence having at least 95%, 96%, 97%, 98%, or 99% identity to the amino acid sequence as set forth in (i) or (ii); or
   consists of the amino acid sequence as set forth in any one of (i) to (iii).
12. The hybrid protein of any one of embodiments 1-11, wherein the human DAF protein is a native human DAF protein or comprises
   (i) the amino acid sequence as set forth in SEQ ID NO: 1;
   (ii) the amino acid sequence encoded by the nucleotide sequence as set forth in SEQ ID NO: 2;
   (iii) the amino acid sequence having at least 95%, 96%, 97%, 98%, or 99% identity to the amino acid sequence as set forth in (i) or (ii); or
   consists of the amino acid sequence as set forth in any one of (i) to (iii).
13. The hybrid protein of any one of embodiments 1-12, wherein
   the CCP1 of human CFH comprises or consists of the amino acid sequence as set forth in SEQ ID NO: 12, 13, 14, or 15, or an amino acid sequence having at least 95%, 96%, 97%, 98%, or 99% identity to the amino acid sequence as set forth in SEQ ID NO: 12, 13, 14, or 15;
   the CCP3 of human DAF comprises or consists of the amino acid sequence as set forth in SEQ ID NO: 7 or 8, or an amino acid sequence having at least 95%, 96%, 97%, 98%, or 99% identity to the amino acid sequence as set forth in SEQ ID NO: 7 or SEQ ID NO: 8;
   the CCP4 of human DAF comprises or consists of the amino acid sequence as set forth in SEQ ID NO: 9, or an amino acid sequence having at least 95%, 96%, 97%, 98%, or 99% identity to the amino acid sequence as set forth in SEQ ID NO: 9; and/or
   the CCP3-4 of human DAF comprises or consists of the amino acid sequence as set forth in SEQ ID NO: 10 or SEQ ID NO: 11, or an amino acid sequence having at least 95%, 96%, 97%, 98%, or 99% identity to the amino acid sequence as set forth in SEQ ID NO: 10 or SEQ ID NO: 11.
14. The hybrid protein of any one of embodiments 1-13, wherein the tag is, for example, a purification tag such as a hexahistidine tag or a biotin tag, comprising, e.g., the amino acid sequence as set forth in SEQ ID NO: 12.
15. The hybrid protein of any one of embodiments 1-14, wherein the signal peptide is secretory, comprising, e.g., the amino acid sequence as set forth in SEQ ID NO: 17.
16. The hybrid protein of any one of embodiments 1-15, wherein the hybrid protein comprises or consists of the amino acid sequence of any one of SEQ ID NOs: 18-33, or an amino acid sequence having at least a 95%, 96%, 97%, 98%, or 99% identity to the amino acid sequence.
17. A nucleic acid molecule encoding the hybrid protein of any one of embodiments 1-16.
18. An expression vector comprising the nucleic acid molecule of embodiment 17, preferably the expression vector is pcDNA3.1.
19. A host cell comprising the nucleic acid molecule of embodiment 17 or the expression vector of embodiment 18, preferably the host cell is prokaryotic or eukaryotic, such as a 293 cell, e.g., an Expi293 cell.
20. A method of preparing the hybrid protein of any one of embodiments 1-16, the method comprising culturing the host cell of embodiment 19 under conditions suitable for expression of the hybrid protein, and optionally further comprising isolating the protein from the host cell or the host cell culture medium, and/or purifying the protein.
21. A pharmaceutical composition or medicament or formulation comprising the hybrid protein according to any one of embodiments 1-16 or a nucleic acid molecule encoding the same, or the nucleic acid molecule of embodiment 17, and optionally a pharmaceutically acceptable excipient.
22. A pharmaceutical combination product comprising
   the hybrid protein according to any one of embodiments 1-16 or a nucleic acid molecule encoding the same, or the nucleic acid molecule of embodiment 17; and the additional therapeutic agents.
23. A method of preventing or treating a complement system-associated disease or disorder in a subject, comprising administering to the subject an effective amount of the hybrid protein of any one of embodiments 1-16 or a nucleic acid molecule encoding the same; or the nucleic acid molecule of embodiment 17; or the pharmaceutical composition or formulation of embodiment 21; or the pharmaceutical combination product of embodiment 22.
24. The method of embodiment 23, wherein the disease or disorder is due to an abnormal activation of the complement system or a dysregulation of the complement system.
25. The method of embodiment 24, wherein the abnormal activation of the complement system or the dysregulation of the complement system is due to for example, a microbial infection or an increase in autoimmune antibodies, or a decrease, deletion, incapacitation, or interference or blockade of a function of complement regulatory proteins.
26. The method of embodiment 23, wherein the complement system-associated disease or disorder is selected from a disease requiring hemolysis inhibition, such as a disease requiring inhibition of hemolysis of the classical pathway of the complement immunity and/or the alternative pathway of the complement immunity; or a disease requiring inhibition of C3b deposition activity, such as dense deposition disease DDD.

### Brief Description of Drawings

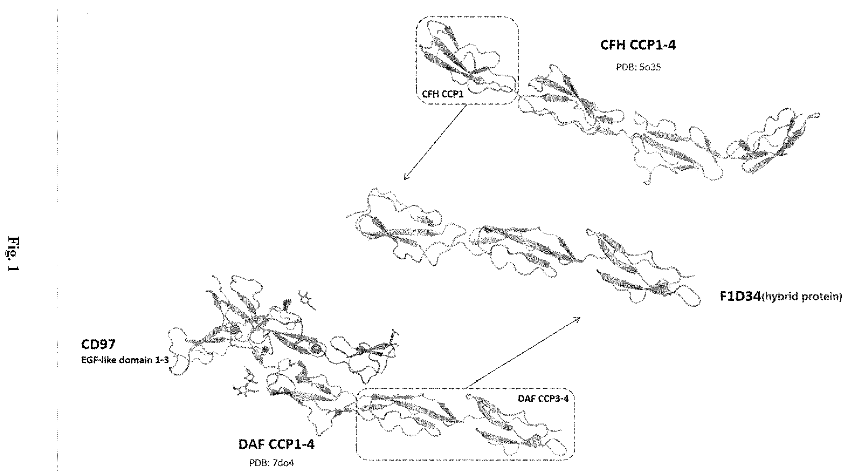

Figure 1 shows the crystal structures of CFH CCP1-5 and DAF CCP1-4 and the structural model of the hybrid protein.
Figure 2 shows the SDS-PAGE electrophoresis pattern of each protein to be tested.
Figure 3 shows the CP inhibitory activity of each protein to be tested.
Figure 4 shows the AP inhibitory activity of each protein to be tested.
Figure 5 shows the C3b deposition inhibitory activity of each protein to be tested.

### Detailed description of the present invention

### I. Definition

It is to be understood that this invention is not limited to the specific methodologies, protocols, and reagents described herein, as these may vary. It should also be understood that the terminology used herein is for describing particular embodiments only, and is not intended to limit the scope of the present invention, which will be limited only by the appended claims. Unless otherwise defined, all technical and scientific terms used herein have the same meaning as commonly understood by one of ordinary skill in the art to which this invention belongs.

### ➢ Decay-accelerating Factor (DAF)

Decay-accelerating Factor (DAF, CD55) is a membrane-associated regulatory protein that protects autologous cells from activation of autologous complement on their surfaces. DAF acts by rapidly dissociating C3 and C5 convertases (central enzymes of the cascade). DAF has the most potent attenuation-accelerating activity of proteins associated with complement regulation and acts on classical pathway enzymes (C4b2a and C4b2a3b) and alternative pathway enzymes (C3bBb and C3BbC3b). However, DAF has no cofactor function.

Structural analysis of the DAF shows that, starting from its N-terminus, it consists of a unit of 4-60 amino acids in length, followed by a fragment (STP) rich in highly O-glycosylated serine (S) and threonine (T), followed by a post-translationally added glycosylphosphatidylinositol (GPI) anchor.

In some embodiments, the DAF is a human DAF having the following accession numbers: Genbank accession numbers M31516, M15799, M64653, S72858, or M643567.

In some embodiments, the DAF is human DAF. In some embodiments, the human DAF is native human DAF. In some embodiments, the amino acid sequence of native human DAF is set forth in SEQ ID NO: 1, with the various modules shown in Table 1 below. Four 60 amino acid long repeating units termed complement control protein repeats (CCP) or Short Consensus Repeats (SCR). CCP1 includes amino acids 35-96; CCP2 includes amino acids 96-160; CCP3 includes amino acids 161-222 and CCP4 includes amino acids 223-285. They provide all the regulatory activities of DAF. The highly O-glycosylated region serves as a cushion that positions the CCP at an appropriate distance suspended above the surface membrane. The GPI anchor allows DAF to move freely on the plasma membrane, making it possible to inactivate invertase complexes anywhere they are assembled. In this context, reference to the amino acid positions of the modules of DAF is made to the amino acid position numbering corresponding to that as set forth in SEQ ID NO: 1.

**Table 1: Protein modules of DAF (divided by UniProt way)**

| The amino acid position of SEQ ID NO:1 | Module |
|---|---|
| 1-34 | Lead sequence |
| 35-96 | CCP1 |
| 96-160 | CCP2 |
| 161-222 | CCP3 |
| 223-285 | CCP4 |
| 287-356 | STP |

In some embodiments, the nucleotide sequence of the cDNA encoding DAF is set forth in SEQ. ID NO: 2.

### ➢ Factor H (CFH or FH)

"Complement Factor H", "Factor H", "FH", "CFH protein" or "CFH" are used interchangeably and refer to a protein of approximately 150 kDa that is a member of the regulators of the complement activation family and is a complement control protein. CFH is a large soluble glycoprotein that circulates in human plasma and serves to regulate the alternative pathway of the complement system, ensuring that the complement system is directed towards pathogens or other dangerous substances and does not damage host tissues.

Factor H is predominantly monomeric but can be weakly self-associated (KD = 28 µM) and may be oligomeric in the presence of glycosaminoglycans or high concentrations of metal ions. CFH consists of 20 homologous units called complement control protein repeats (CCPs) (SCR or sushi domains), some of which function for cell attachment, while other repeats function to eliminate C3b from the cell surface. Each of the 20 SCRs is about 60 amino acids in length, arranged head to tail, comprising 4 cysteine residues, forming 2 disulfide bonds for each module. SCR 19 and 20 are involved in C3b binding.

The splice variant FHL-1 consists of the first 7 CCPs, followed by the C-terminal sequence Ser-Pro-Leu-Thr. Each CCP comprises approximately 60 residues, including four invariant cysteines, forming Cys^{I}-Cys^{III}, Cys^{II}-Cys^{IV} disulfides. Adjacent modules are connected by sequences of three to eight residues.

The images from negative staining electron microscopy show that FH molecules adopt a variety of conformations, but are mainly folded in half; analytical ultracentrifugation, small angle X-ray scattering (deposition of the module in PDB, e.g., 3 GAV), and chemical cross-linking also indicated that the module chain itself was bent. The following high resolution structure of several FH fragments was determined, alone or in complex with other molecules (PDB identifier is provided in parenthesis below): CCP 1-2 (2RLP), 2-3 (2RLQ), 1-4 (2WII), 5, 6-7 (e.g., 2W80, 2 YBY), 7 (2JGW and 2JGX), 6-8 (2 UWN and 2V8E), 9 (4K12), 10-11 (4B2R), 11-12 (4B2S), 12-13 (2KMS), 15 (1HFI), 16 (1HCC), 15-16 (1HFH), 18-20 (3SWO), 19-20 (e.g., 2BZM, 2G7I and 4ONT). Each CCP resembles a prolate spheroid having a major axis of about 4 nm and a minor axis of about 2 nm and comprises beta strands in anti-parallel patches that are approximately aligned with the major axis, with the N- and C-termini located at either end of its long axis, facilitating an end-to-end arrangement of tandem CCPs with variable inter-module contact, tilt, and twists.

In some embodiments, the CFH is a human CFH, e.g., a native human CFH. In some embodiments, the amino acid sequence of CFH has the following accession numbers: HGNC: HGNC: 4883, Ensembl: ENSG00000000971, HPRD: 00601, MIM: 134370 or Vega: OTTHUMG00000035607.

In some embodiments, the amino acid sequence of CFH is set forth in SEQ ID NO: 3. In some embodiments, the amino acid positions corresponding to each module of the CFH are shown in Table 2 below.

**Table 2: Protein modules of CFH (divided by UniProt way)**

| The amino acid position of SEQ ID NO: 3 | Module |
|---|---|
| 1-18 | Lead peptide |
| 19-82 | CCP1 |
| 83-143 | CCP2 |
| 144-207 | CCP3 |
| 208-264 | CCP4 |
| 265-322 | CCP5 |
| 324-386 | CCP6 |
| 387-444 | CCP7 |
| 446-507 | CCP8 |
| 515-566 | CCP9 |
| 567-625 | CCP10 |
| 628-686 | CCP11 |
| 689-746 | CCP12 |
| 751-805 | CCP13 |
| 809-866 | CCP14 |
| 868-928 | CCP15 |
| 929-986 | CCP16 |
| 987-1045 | CCP17 |
| 1046-1104 | CCP18 |
| 1107-1165 | CCP19 |
| 1170-1230 | CCP20 |

In some embodiments, the nucleotide sequence of the cDNA encoding CFH is set forth in SEQ. ID NO: 4.

In some embodiments, the protein sequence encoding the splice variant FHL-1 of CFH is set forth in SEQ ID NO: 5. In some embodiments, the nucleotide sequence of the cDNA encoding the splice variant FHL-1 of CFH is set forth in SEQ ID NO: 6.

In this context, reference to the amino acid positions of the modules of CFH is made to the amino acid position numbering corresponding to that as set forth in SEQ ID NO: 3.

### ➢ Other definitions

For the purpose of interpreting this specification, the following definitions will be used and, wherever appropriate, terms used in the singular may also include the plural and vice versa. It is to be understood that the terminology used herein is for the purpose of describing particular embodiments only and is not intended to be limiting.

The term "about" when used in conjunction with a numerical value is intended to encompass a range of numerical values having a lower limit that is 5% less than the specified numerical value and an upper limit that is 5% greater than the specified numerical value.

As used herein, the term "and/or" means any one of the options or two or more or all of the options.

As used herein, the term "comprises" or "includes" means the inclusion of the stated element, integer, or step, but not the exclusion of any other element, integer, or step. When the term "comprises" or "includes" is used herein, it also encompasses a combination of the stated elements, integers, or steps unless otherwise indicated. For example, reference to a protein "comprising" a specific sequence is intended to also encompass the protein consisting of that specific sequence.

As used herein, the terms "hybrid protein" and "chimeric polypeptide" are used interchangeably and refer to a larger polypeptide formed by the fusion of at least two heterologous polypeptide sequences, optionally through a linker. The hybrid proteins can be produced by recombinant expression.

"Percent identity (%)" of an amino acid sequence refers to the percentage of amino acid residues in a candidate sequence that are identical to the amino acid residues of the specific amino acid sequence shown in this specification, after aligning the candidate sequence with the specific amino acid sequence shown in this specification and introducing gaps, if necessary, to achieve the maximum percent sequence identity, and without taking into account any conservative substitutions included as part of sequence identity. In some embodiments, the present invention contemplates variants of a protein or polypeptide of the present invention that have a substantial degree of identity, e.g., at least 80%, 85%, 90%, 95%, 97%, 98%, or 99% or more identity, relative to the polypeptide or protein specifically disclosed herein. The variants may comprise conservative changes.

For polypeptide sequences, "conservative alterations" include substitutions, deletions, or additions to the polypeptide sequence that do not substantially change the desired functional activity of the polypeptide sequence. For example, conservative substitutions often result in the replacement of an amino acid with a chemically similar amino acid. Conservative substitution tables providing functionally similar amino acids are well known in the art. 8 groups of amino acids conservatively substituted for each other are listed as follows: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M) ), Valine (V); 6) Phenylalanine (F), Tyrosine (Y), Tryptophan (W); 7) Serine (S), Threonine (T); and 8) Cysteine (C), Methionine (M). In some embodiments, the term "conservative sequence alteration" is used to refer to amino acid modifications that do not significantly affect or alter the activity of the parent hybrid protein. For example, conservatively modified variants retain at least 80%, 85%, 90%, 95%, 98%, 99%, or more, e.g., 100-110% or more activity relative to the parent polypeptide or hybrid protein.

The term "host cell" refers to a cell into which an exogenous polynucleotide has been introduced, including the progeny of such cells. Host cells include "transformants" and "transformed cells," which include primary transformed cells and progeny derived therefrom. Host cells are any type of cell system that can be used to produce hybrid proteins of the present invention, including eukaryotic cells, such as mammalian cells, insect cells, yeast cells; and prokaryotic cells, such as E. coli cells. Host cells include cultured cells, as well as cells within transgenic animals, transgenic plants, or cultured plant tissue or animal tissue.

The term "expression vector" refers to a vector comprising a recombinant polynucleotide comprising expression control sequences operably linked to the nucleotide sequence to be expressed. The expression vector comprises sufficient cis-acting elements for expression; other elements for expression can be provided by the host cell or in an in vitro expression system. Expression vectors include all those known in the art, including cosmids, plasmids (e.g., naked or included in liposomes), and viruses (e.g., lentiviruses, retroviruses, adenoviruses, viruses, and adeno-associated viruses) into which recombinant polynucleotides are incorporated.

The terms "individual" or "subject" are used interchangeably and refer to a mammal. Mammals include, but are not limited to, domesticated animals (e.g., cows, sheep, cats, dogs, and horses), primates (e.g., humans and non-human primates such as monkeys), rabbits, and rodents (e.g., mice and rats)). In particular, individuals are people.

The term "treating" includes administering a composition or hybrid polypeptide to prevent or delay the onset of symptoms, complications, or biochemical indication of a disease, alleviate symptoms, or prevent or inhibit further progression of the disease, condition, or disorder. The term "prevention" includes the inhibition of the occurrence or progression of a disease or disorder or symptoms of a particular disease or disorder.

The term "pharmaceutical excipient" refers to diluents, adjuvants (such as Freund's adjuvant (complete and incomplete)), excipients, carriers or stabilizers, etc., which are administered with the active substance.

The term "pharmaceutical composition" refers to a composition that is in a form effective to permit the biological activity of the active ingredients comprised therein and does not comprise additional ingredients that would have unacceptable toxicities to the subject to whom the composition is administered.

The term "effective amount" refers to an amount or dosage of a hybrid protein of the present invention or a nucleic acid encoding the same or a composition or combination thereof that, when administered to a patient in single or multiple doses, produces the desired effect in a patient in need of treatment or prevention.

A "therapeutically effective amount" refers to an amount effective to achieve the desired therapeutic result, at the required doses and for the required period of time. A therapeutically effective amount is also an amount in which any toxic or detrimental effects of the hybrid protein or composition or combination are outweighed by the therapeutically beneficial effects. A "therapeutically effective amount" preferably inhibits a measurable parameter or improves a measurable parameter by at least about 40%, even more preferably at least about 50%, 55%, 60%, 65%, 70%, 75%, 80%, 85%, 90% or even 100%, relative to an untreated subject.

A "prophylactically effective amount" refers to an amount effective to achieve the desired prophylactic result, at the required dosage and for the required period of time. Generally, the prophylactically effective amount will be less than the therapeutically effective amount because the prophylactic dose is administered in the subject before or at an earlier stage of the disease.

These and other aspects and embodiments of the present invention are described in the accompanying drawings (Brief Description of the Drawings immediately follows) and the following detailed description of the present invention and are illustrated in the following examples. Any or all features discussed above and throughout this application may be combined in various embodiments of the present invention. The following examples further illustrate the present invention, however, it is to be understood that the examples are described by way of illustration rather than limitation and that various modifications may be made by those skilled in the art.

### II. Hybrid protein

The present invention relates to a hybrid protein of CFH and DAF, which comprises at least one functional unit derived from CFH and at least one functional unit derived from DAF.

### A. DAF functional unit

In certain embodiments, the hybrid protein of the present invention preferably comprises a functional unit from DAF. Such functional units are capable of dissociating the C3 and C5 convertases and/or accelerating the decay-accelerating activity against C3 convertases in the classical pathway and/or in the alternative pathway. In some embodiments, the DAF functional unit comprises CCPs 3 and 4 of the DAF.

The amino acid sequence of such CCPs can be identical to the native or naturally occurring amino acid sequence of DAF. Alternatively, the amino acid sequence of such CCPs may be slightly altered, particularly at the amino or carboxy terminus. This alteration occurs when the restriction enzyme site is incorporated into the polynucleotide encoding the CCP. Such alterations also occur when an amino acid is deleted from the N-terminus or C-terminus of the functional unit. For example, in some embodiments, 1-2 amino acids may be deleted at the N-terminus of CCP3.

Some amino acid substitutions, preferably conservative substitutions, in the sequence may also be introduced without affecting functional activity. Conservative substitutions may be made, for example, by substituting charged amino acids for one another or substituting hydrophilic amino acids for one another, hydrophobic amino acids for one another, and amino acids of similar mass for one another.

In one embodiment, the DAF functional unit comprises an amino acid sequence that is at least 95%, 96%, 97%, 98%, or 99% identical to CCP3 and/or 4 of the human DAF protein (or CCP3-4 directly linked together by the C-terminus of CCP3 and the N-terminus of CCP4). In some embodiments, the DAF functional unit comprises CCP3 and/or 4 of the human DAF protein (CCP3-4 directly linked together by the C-terminus of CCP3 and the N-terminus of CCP4). In some embodiments, the DAF functional unit comprises CCP3 and 4 of the human DAF protein. In some embodiments, the DAF function unit is CCP3-4, which is directly linked together by the C-terminus of CCP3 and the N-terminus of CCP4.

In some embodiments, the human DAF protein is a native human DAF protein. In some embodiments, the native human DAF protein comprises or consists of the amino acid sequence shown as SEQ ID NO: 1. DAF comprises or consists of the amino acid sequence encoded by the DNA sequence as set forth in SEQ ID NO: 2.

In some embodiments, CCP3 comprises or consists of amino acids 161-222 of DAF. In some embodiments, 1-2 amino acids of the N-terminus of CCP3 may be deleted for attachment of other functional units, e.g., CCP3 comprises or consists of amino acids 163-222 of DAF.

In some embodiments, CCP3 comprises or consists of the amino acid sequence as set forth in SEQ ID NO: 7 or 8, or an amino acid sequence having at least 95%, 96%, 97%, 98%, or 99% identity to the amino acid sequence as set forth in SEQ ID NO: 7 or SEQ ID NO: 8.
SEQ ID NO: 7: 
KSCPNPGEIRNGQIDVPGGILFGATISFSCNTGYKLFGSTSSFCLISGSSVQWSD
PLPECRE
SEQ ID NO: 8:

CPNPGEIRNGQIDVPGGILFGATISFSCNTGYKLFGSTSSFCLISGSSVQWSDPLP
ECRE.

In some embodiments, CCP4 comprises or consists of amino acids 223-285 of DAF.

In some embodiments, CCP4 comprises or consists of the amino acid sequence as set forth in SEQ ID NO: 9, or an amino acid sequence having at least 95%, 96%, 97%, 98%, or 99% identity to the amino acid sequence as set forth in SEQ ID NO: 9.

SEQ ID NO: 9:

IYCPAPPQIDNGIIQGERDHYGYRQSVTYACNKGFTMIGEHSIYCTVNNDEGE
WSGPPPECRG

In some embodiments, CCP3-4 comprises or consists of amino acids 161-285 or amino acids 163-285 of DAF. In some embodiments, the DAF functional unit CCP3-4 comprises or consists of the amino acid sequence as set forth in SEQ ID NO: 10 or SEQ ID NO: 11, or an amino acid sequence having at least 95%, 96%, 97%, 98%, or 99% identity to the amino acid sequence as set forth in SEQ ID NO: 10 or SEQ ID NO: 11.
SEQ ID NO: 10: Human DAF CCP3-4 
KSCPNPGEIRNGQIDVPGGILFGATISFSCNTGYKLFGSTSSFCLISGSSVQWSD
PLPECREIYCPAPPQIDNGIIQGERDHYGYRQSVTYACNKGFTMIGEHSIYCTV
NNDEGEWSGPPPECRG
SEQ ID NO: 11: Human DAF CCP3-4 (2 amino acids are missing from the N-terminus of CCP3)

CPNPGEIRNGQIDVPGGILFGATISFSCNTGYKLFGSTSSFCLISGSSVQWSDPLP
ECREIYCPAPPQIDNGIIQGERDHYGYRQSVTYACNKGFTMIGEHSIYCTVNND
EGEWSGPPPECRG

When referring to the amino acid position of DAF, the amino acid sequence position corresponds to the amino acid position of SEQ ID NO: 1.

### B. CFH functional unit

In certain embodiments, the hybrid protein of the present invention comprises functional units from CFH. This functional unit is capable of dissociating the alternative pathway C3 convertase C3bBb and/or binding to C3b. In a preferred embodiment, the CFH functional unit comprises CCP1 of CFH.

The amino acid sequence of the CCP of such CFH may be identical to the native or naturally occurring amino acid sequence of CFH. Alternatively, the amino acid sequence of such CCPs may be slightly altered, particularly at the amino or carboxy terminus. This change occurs when the restriction enzyme site is incorporated into the polynucleotide encoding the CCP. Such changes also occur when amino acids are deleted from or added to the N-terminus or C-terminus of the functional unit.

For example, in some embodiments, 1-2 amino acids may be deleted at the C-terminus of CCP1.

In some embodiments, 1-2 amino acids of CCP2 can also be added to the C-terminus of CCP1 to attach functional units of DAF, thus, "CCP1" as described herein encompasses the CCP1 with amino acids (e.g., 1-2 amino acids of CCP2) added at the C-terminus. For example, in the case where 1-2 amino acids are deleted from the N-terminus of the functional unit of DAF, 1-2 amino acids of CCP2 may be added to the C-terminus of CCP1 for linking the functional unit of DAF. In some embodiments, KS are deleted from the CCP3 of DAF, and RP are added to the C-terminus of CCP1 of CFH, both of which are linked to obtain a hybrid protein.

Some amino acid substitutions, preferably conservative substitutions, in the sequence, may also be introduced without affecting functional activity. Conservative substitutions may be made, for example, by substituting charged amino acids for one another or substituting hydrophilic amino acids for one another, hydrophobic amino acids for one another, and amino acids of similar mass for one another. For example, CCP1 of the CFH of the present invention can comprise a V62I mutation.

In one embodiment, the CFH functional unit comprises an amino acid sequence having at least 95%, 96%, 97%, 98%, or 99% identity to CCP1 of the human CFH protein. In some embodiments, the CFH functional unit comprises CCP1 of the human CFH protein.

In some embodiments, the human CFH protein is a native human CFH protein. In some embodiments, the native human CFH protein comprises or consists of an amino acid sequence as set forth in SEQ ID NO: 3 or 5. In some embodiments, the CFH comprises or consists of the amino acid sequence encoded by the DNA sequence as set forth in SEQ ID NO: 4 or 6.

In some embodiments, CCP1 comprises or consists of amino acids 19-82 of CFH. In some embodiments, CCP1 comprises or consists of amino acids 19-to 84 of CFH.

In some embodiments, the CFH functional unit comprises or consists of the amino acid sequence as set forth in SEQ ID NO: 12, 13, 14, or 15, or an amino acid sequence having at least 95%, 96%, 97%, 98%, or 99% identity to the amino acid sequence as set forth in SEQ ID NO: 12, 13, 14, or 15.
SEQ ID NO: 12: Human CFH CCP1 (the C-terminus of CCP1 is added with two N-terminal amino acids RP of CCP2) 
EDCNELPPRRNTEILTGSWSDQTYPEGTQAIYKCRPGYRSLGNVIMVCRK
GEWVALNPLRKCQKRP
SEQ ID NO: 13: Human CFH CCP1 (CCP1) 
EDCNELPPRRNTEILTGSWSDQTYPEGTQAIYKCRPGYRSLGNVIMVCRK
GEWVALNPLRKCQK
SEQ ID NO: 14: Human CFH CCP1 (the C-terminus of CCP1 is added with two N-terminal amino acids RP of CCP2 and comprises V62I) 
EDCNELPPRRNTEILTGSWSDQTYPEGTQAIYKCRPGYRSLGNIIMVCRK
GEWVALNPLRKCQKRP
SEQ ID NO:15: Human CFH CCP1 (CCP1 comprises V62I)

EDCNELPPRRNTEILTGSWSDQTYPEGTQAIYKCRPGYRSLGNIIMVCRKGEW
VALNPLRKCQK

When referring to the amino acid position of CFH, the amino acid sequence position corresponds to the amino acid position of SEQ ID NO: 3.

### C. Other units

Optionally, the hybrid protein of the present invention may further comprise a tag, preferably of about 2-10 amino acids, added to the amino or carboxy terminus, e.g. the carboxy terminus, of the hybrid protein. Typically, such additions are made to stabilize the protein or to facilitate secretory expression or purification of the hybrid protein. Such tags are known in the art. Representative examples of such tags include sequences encoding a series of histidine residues (e.g., 2-10 histidines, e.g., 2, 3, 4, 5, 6, or 7 histidines), an epitope tag FLAG, herpes simplex glycoprotein D, beta-galactosidase, a maltose-binding protein, or glutathione S-transferase.

In some embodiments, the tag is a histidine residue, which may be added at the amino-terminus or the carboxy-terminus, e.g., the carboxy-terminus, of the hybrid protein.

In some embodiments, the tag is a 6×His tag of GHHHHHH (SEQ ID NO: 16).

Optionally, the hybrid protein of the present invention may also include a signal peptide, such as MGWSCIILFLVATATGVHS (SEQ ID NO: 17).

The present invention also encompasses hybrid proteins in which one or more amino acids are altered by post-translational processes or synthetic methods. Examples of such modifications include, but are not limited to, glycosylation, iodination, myristoylation, and PEGylation.

### D. Examples of hybrid proteins

In some embodiments, the hybrid protein of the present invention comprises at least one functional unit from CFH and at least one functional unit from DAF.

In some embodiments, the hybrid protein of the present invention comprises CCP1 of CFH and CCP3 and CCP4 of DAF. In some embodiments, the hybrid protein of the present invention consists of CCP1 of CFH and CCP3 and CCP4 of DAF. In some embodiments, the hybrid protein of the present invention comprises or consists of CCP1 of CFH and CCP3-4 of DAF.

In some embodiments, 1-2 amino acids (e.g., 1-2 amino acids at the N-terminus of CCP2) are added to the C-terminus of CCP1 of CFH. In some embodiments, 1-2 amino acids are deleted from the N-terminus of CCP3 or CCP3-4 of DAF.

In some embodiments, the hybrid protein of the present invention comprises CCP1 of CFH and CCP3-4 of DAF, wherein 1-2 N-terminal amino acids of CCP2 are added to the C-terminus of CCP1 and 1-2 amino acids are deleted correspondingly from the N-terminus of CCP3 of DAF. In some specific embodiments, the hybrid protein of the present invention comprises CCP1 of CFH and CCP3-4 of DAF, wherein the N-terminal amino acid RP of CCP2 are added to the C-terminus of CCP1 and the amino acid KS are deleted correspondingly from the N-terminus of CCP3 of DAF.

In some embodiments, CCP1 of the CFH of the present invention can comprise a V62I mutation.

In some embodiments, the hybrid protein of the present invention further comprises a signal peptide at the N-terminus, such as the amino acid sequence as set forth in SEQ ID NO: 13, and/or a tag at the C-terminus, such as a histidine tag, such as a 6×His-tag, such as GHHHHHH.

In some embodiments, CCP3 of DAF comprises or consists of amino acids 161-222 of the DAF protein and/or CCP4 comprises or consists of amino acids 223-285 of the DAF protein, wherein the amino acid positions are numbered corresponding to the amino acid positions as set forth in SEQ ID NO: 1. In some embodiments, the CCP3 of DAF is deleted by 2 amino acids from its N-terminus, for example comprising or consisting of amino acids 163-222 of the DAF protein, wherein said amino acid positions are numbered corresponding to the amino acid positions as set forth in SEQ ID NO: 1. In some embodiments, the CCP3-4 of the DAF comprises or consists of amino acids 161-285 of the DAF protein, wherein the amino acid positions are numbered corresponding to the amino acid positions as set forth in SEQ ID NO: 1. In some embodiments, the CCP3-4 of DAF is deleted at its N-terminus by 2 amino acids, for example comprising or consisting of amino acids 163-285 of the DAF protein, wherein said amino acid positions are numbered corresponding to the amino acid positions as set forth in SEQ ID NO: 1.

In some embodiments, CCP1 of CFH comprises or consists of amino acids 19-82 of the CFH protein, wherein the amino acid positions are numbered corresponding to the amino acid positions as set forth in SEQ ID NO: 3. In some embodiments, CCP1 of CFH comprises or consists of amino acids 19-84 of the CFH protein, wherein the amino acid positions are numbered corresponding to the amino acid positions as set forth in SEQ ID NO: 3.

In some embodiments, CCP3-4 of DAF comprises or consists of amino acids 161-285 of the DAF protein, wherein the amino acid positions are numbered corresponding to the amino acid positions as set forth in SEQ ID NO: 1; and CCP1 of CFH comprises or consists of amino acids 19-82 of the CFH protein, wherein the amino acid positions are numbered corresponding to the amino acid positions as set forth in SEQ ID NO: 3.

In some embodiments, CCP3-4 of DAF comprises or consists of amino acids 163-285 of the DAF protein, wherein the amino acid positions are numbered corresponding to the amino acid positions as set forth in SEQ ID NO: 1; and CCP1 of CFH comprises or consists of amino acids 19-84 of the CFH protein, wherein the amino acid positions are numbered corresponding to the amino acid positions as set forth in SEQ ID NO: 3.

In some specific embodiments, the DAF is a human DAF protein, such as a native human DAF protein. In some specific embodiments, the DAF protein comprises or consists of
(i) the amino acid sequence as set forth in SEQ ID NO: 1;
(ii) the amino acid sequence encoded by the nucleotide sequence as set forth in SEQ ID NO: 2;
(iii) the amino acid sequence having at least 95%, 96%, 97%, 98%, or 99% identity to the amino acid sequence as set forth in (i) or (ii); or
consists of the amino acid sequence as set forth in any one of (i) to (iii).

In some specific embodiments, CFH is a human CFH protein, such as a native human CFH protein. In some specific embodiments, the CFH protein comprises
(i) the amino acid sequence as set forth in SEQ ID NO: 3 or 5;
(ii) the amino acid sequence encoded by the nucleotide sequence as set forth in SEQ ID NO: 4 or 6;
(iii) the amino acid sequence having at least 95%, 96%, 97%, 98%, or 99% identity to the amino acid sequence as set forth in (i) or (ii); or
consists of the amino acid sequence as set forth in any one of (i) to (iii).

In some embodiments, the hybrid protein of the present invention comprises or consists of the amino acid sequence of any one of SEQ ID NOs: 18-21, or an amino acid sequence having at least 95%, 96%, 97%, 98%, or 99% identity to the amino acid sequence.
> Human CFH CCP1 or SCR1 - Human DAF/CD55 CCP3-4 or SCR3-4 (in bold): 
EDCNELPPRRNTEILTGSWSDQTYPEGTQAIYKCRPGYRSLGNVIMVCRKGE
WVALNPLRKCQKRPCPNPGEIRNGQIDVPGGILFGATISFSCNTGYKLFGST
SSFCLISGSSVQWSDPLPECREIYCPAPPQIDNGIIQGERDHYGYRQSVTYA
CNKGFTMIGEHSIYCTVNNDEGEWSGPPPECRG (SEQ ID NO: 18)
 
EDCNELPPRRNTEILTGSWSDQTYPEGTQAIYKCRPGYRSLGNVIMVCRKGE
WVALNPLRKCQKKSCPNPGEIRNGQIDVPGGILFGATISFSCNTGYKLFGST
SSFCLISGSSVQWSDPLPECREIYCPAPPQIDNGIIQGERDHYGYRQSVTYA
CNKGFTMIGEHSIYCTVNNDEGEWSGPPPECRG (SEQ ID NO: 19)
>Human CFH CCP1 or SCR1 - Human DAF/CD55 CCP3-4 or SCR3-4 (in bold), comprising a V62I mutation:

EDCNELPPRRNTEILTGSWSDQTYPEGTQAIYKCRPGYRSLGNIIMVCRKGEW
VALNPLRKCQKKSCPNPGEIRNGQIDVPGGILFGATISFSCNTGYKLFGSTS
SFCLISGSSVQWSDPLPECREIYCPAPPQIDNGIIQGERDHYGYRQSVTYAC
NKGFTMIGEHSIYCTVNNDEGEWSGPPPECRG (SEQ ID NO: 20)

EDCNELPPRRNTEILTGSWSDQTYPEGTQAIYKCRPGYRSLGNIIMVCRKGEW
VALNPLRKCQKRPCPNPGEIRNGQIDVPGGILFGATISFSCNTGYKLFGSTS
SFCLISGSSVQWSDPLPECREIYCPAPPQIDNGIIQGERDHYGYRQSVTYAC
NKGFTMIGEHSIYCTVNNDEGEWSGPPPECRG (SEQ ID NO: 21)

In some embodiments, the hybrid protein of the present invention comprises or consists of the amino acid sequence according to any one of SEQ ID NOs: 22-25, or an amino acid sequence having at least 95%, 96%, 97%, 98%, or 99% identity to the amino acid sequence.
> Human CFH CCP1 or SCR1 - Human DAF/CD55 CCP3-4 or SCR3-4 (in bold) - 6×His (italics): 
EDCNELPPRRNTEILTGSWSDQTYPEGTQAIYKCRPGYRSLGNVIMVCRKGE
WVALNPLRKCQKRPCPNPGEIRNGQIDVPGGILFGATISFSCNTGYKLFGST
SSFCLISGSSVQWSDPLPECREIYCPAPPQIDNGIIQGERDHYGYRQSVTYA
CNKGFTMIGEHSIYCTVNNDEGEWSGPPPECRGGHHHHHH (SEQ ID NO:
22)
 
EDCNELPPRRNTEILTGSWSDQTYPEGTQAIYKCRPGYRSLGNVIMVCRKGE
WVALNPLRKCQKKSCPNPGEIRNGQIDVPGGILFGATISFSCNTGYKLFGST
SSFCLISGSSVQWSDPLPECREIYCPAPPQIDNGIIQGERDHYGYRQSVTYA
CNKGFTMIGEHSIYCTVNNDEGEWSGPPPECRGGHHHHHH (SEQ ID NO:
23)
>Human CFH CCP1 or SCR1 - Human DAF/CD55 CCP3-4 or SCR3-4 (in bold) - 6×His (italics):
   comprising a V62I mutation:

EDCNELPPRRNTEILTGSWSDQTYPEGTQAIYKCRPGYRSLGNIIMVCRKGEW
VALNPLRKCQKKSCPNPGEIRNGQIDVPGGILFGATISFSCNTGYKLFGSTS
SFCLISGSSVQWSDPLPECREIYCPAPPQIDNGIIQGERDHYGYRQSVTYAC
NKGFTMIGEHSIYCTVNNDEGEWSGPPPECRGGHHHHHH (SEQ ID NO:
24)

EDCNELPPRRNTEILTGSWSDQTYPEGTQAIYKCRPGYRSLGNIIMVCRKGEW
VALNPLRKCQKRPCPNPGEIRNGQIDVPGGILFGATISFSCNTGYKLFGSTS
SFCLISGSSVQWSDPLPECREIYCPAPPQIDNGIIQGERDHYGYRQSVTYAC
NKGFTMIGEHSIYCTVNNDEGEWSGPPPECRGGHHHHHH (SEQ ID NO:
25)

In some embodiments, the hybrid protein of the present invention comprises or consists of the amino acid sequence according to any one of SEQ ID NOs: 26-29, or an amino acid sequence having at least 95%, 96%, 97%, 98%, or 99% identity to the amino acid sequence.
>Signal peptide - human CFH CCP1 or SCR1 - human DAF/CD55 CCP3-4 or SCR3-4 (in bold) - 6×His (italic): 
MGWSCIILFLVATATGVHSEDCNELPPRRNTEILTGSWSDQTYPEGTQAIYKCR
PGYRSLGNVIMVCRKGEWVALNPLRKCQKRPCPNPGEIRNGQIDVPGGILF
GATISFSCNTGYKLFGSTSSFCLISGSSVQWSDPLPECREIYCPAPPQIDNGI
IQGERDHYGYRQSVTYACNKGFTMIGEHSIYCTVNNDEGEWSGPPPECR
GGHHHHHH (SEQ ID NO:26)
 
MGWSCIILFLVATATGVHSEDCNELPPRRNTEILTGSWSDQTYPEGTQAIYKCR
PGYRSLGNVIMVCRKGEWVALNPLRKCQKKSCPNPGEIRNGQIDVPGGILF
GATISFSCNTGYKLFGSTSSFCLISGSSVQWSDPLPECREIYCPAPPQIDNGI
IQGERDHYGYRQSVTYACNKGFTMIGEHSIYCTVNNDEGEWSGPPPECR
GGHHHHHH (SEQ ID NO:27)
>Signal peptide - human CFH CCP1 or SCR1 - human DAF/CD55 CCP3-4 or SCR3-4 (in bold) - 6×His (italic):
   comprising a V62I mutation:

MGWSCIILFLVATATGVHSEDCNELPPRRNTEILTGSWSDQTYPEGTQAIYKCR
PGYRSLGNIIMVCRKGEWVALNPLRKCQKKSCPNPGEIRNGQIDVPGGILFG
ATISFSCNTGYKLFGSTSSFCLISGSSVQWSDPLPECREIYCPAPPQIDNGII
QGERDHYGYRQSVTYACNKGFTMIGEHSIYCTVNNDEGEWSGPPPECRG
GHHHHHH (SEQ ID NO: 28)

MGWSCIILFLVATATGVHSEDCNELPPRRNTEILTGSWSDQTYPEGTQAIYKCR
PGYRSLGNIIMVCRKGEWVALNPLRKCQKRPCPNPGEIRNGQIDVPGGILFG
ATISFSCNTGYKLFGSTSSFCLISGSSVQWSDPLPECREIYCPAPPQIDNGII
QGERDHYGYRQSVTYACNKGFTMIGEHSIYCTVNNDEGEWSGPPPECRG

GHHHHHH (SEQ ID NO: 29)

In some embodiments, the hybrid protein of the present invention comprises or consists of the amino acid sequence according to any one of SEQ ID NOs: 30-33, or an amino acid sequence having at least 95%, 96%, 97%, 98%, or 99% identity to the amino acid sequence.
>Signal peptide - human CFH CCP1 or SCR1 - human DAF/CD55 CCP3-4 or SCR3-4 (in bold): 
MGWSCIILFLVATATGVHSEDCNELPPRRNTEILTGSWSDQTYPEGTQAIYKCR
PGYRSLGNVIMVCRKGEWVALNPLRKCQKRPCPNPGEIRNGQIDVPGGILF
GATISFSCNTGYKLFGSTSSFCLISGSSVQWSDPLPECREIYCPAPPQIDNGI
IQGERDHYGYRQSVTYACNKGFTMIGEHSIYCTVNNDEGEWSGPPPECR
G (SEQ ID NO: 30)
 
MGWSCIILFLVATATGVHSEDCNELPPRRNTEILTGSWSDQTYPEGTQAIYKCR
PGYRSLGNVIMVCRKGEWVALNPLRKCQKKSCPNPGEIRNGQIDVPGGILF
GATISFSCNTGYKLFGSTSSFCLISGSSVQWSDPLPECREIYCPAPPQIDNGI
IQGERDHYGYRQSVTYACNKGFTMIGEHSIYCTVNNDEGEWSGPPPECR
G (SEQ ID NO: 31)
>Signal peptide - human CFH CCP1 or SCR1 - human DAF/CD55 CCP3-4 or SCR3-4 (in bold):
   comprising a V62I mutation:

MGWSCIILFLVATATGVHSEDCNELPPRRNTEILTGSWSDQTYPEGTQAIYKCR
PGYRSLGNIIMVCRKGEWVALNPLRKCQKKSCPNPGEIRNGQIDVPGGILFG
ATISFSCNTGYKLFGSTSSFCLISGSSVQWSDPLPECREIYCPAPPQIDNGII
QGERDHYGYRQSVTYACNKGFTMIGEHSIYCTVNNDEGEWSGPPPECRG
(SEQ ID NO: 32)

MGWSCIILFLVATATGVHSEDCNELPPRRNTEILTGSWSDQTYPEGTQAIYKCR
PGYRSLGNIIMVCRKGEWVALNPLRKCQKRPCPNPGEIRNGQIDVPGGILFG
ATISFSCNTGYKLFGSTSSFCLISGSSVQWSDPLPECREIYCPAPPQIDNGII
QGERDHYGYRQSVTYACNKGFTMIGEHSIYCTVNNDEGEWSGPPPECRG
(SEQ ID NO: 33)

### III. Polynucleotides, vectors and hosts

The present invention provides a nucleic acid encoding any of the above hybrid proteins of the present invention. Also provided is a vector comprising the nucleic acid. In one embodiment, the vector is an expression vector (e.g., a pcDNA vector, such as pcDNA3.1). Also provided is a host cell comprising the nucleic acid or the vector. In one embodiment, the host cell is eukaryotic. In another embodiment, the host cell is selected from a yeast cell or a mammalian cell (e.g., CHO cells or 293 cells, such as Expi293 cells). In another embodiment, the host cell is prokaryotic.

In one aspect, the present invention provides a nucleic acid encoding any of the above hybrid proteins. The polypeptide encoded by the nucleic acid is capable of having DAF and/or CFH function when expressed from a suitable expression vector, e.g., dissociating C3 and/or C5 convertase, accelerating decay-accelerating activity against the classical pathway C3 convertase and/or the alternative pathway C3 convertase, dissociating the alternative pathway C3 convertase C3bBb, or binding to C3b, and complement-inhibiting activity, including activity inhibiting the Alternative Pathway (AP), the Classical Pathway (CP), and/or C3b deposition.

For ease of production and purification, the hybrid protein may be fused at the N-terminus to a secretory signal peptide, and/or a tag peptide, such as a hexahistidine tag or biotin tag, to facilitate the purification.

As will be appreciated by those skilled in the art, each hybrid protein may be encoded by a variety of nucleic acid sequences due to codon degeneracy.

In some embodiments, the nucleic acid of the present invention comprises a nucleic acid encoding the amino acid sequence selected from any one of SEQ ID NOs: 18-33, or an amino acid sequence having at least 85%, 90%, 91%, 92%, 93%, 94%, 95%, 96%, 97%, 98%, or 99% identity to the amino acid sequence selected from any one of SEQ ID NOs: 18-33.

Nucleic acid sequences encoding the molecules of the present invention may be generated using methods well known in the art, for example by de novo solid-phase DNA synthesis, or by PCR amplification.

In one embodiment, one or more vectors comprising a nucleic acid of the present invention are provided. In one embodiment, the vector is an expression vector, such as a prokaryotic expression vector or a eukaryotic expression vector. Vectors include, but are not limited to, viruses, plasmids, cosmids, lambda phages, or Yeast Artificial Chromosomes (YACs). In a preferred embodiment, the expression vector is a pcDNA, such as pcDNA3.1.

In one embodiment, a host cell comprising one or more polynucleotides of the present invention is provided. In some embodiments, host cells comprising the expression vectors of the present invention are provided. As used herein, the term "host cell" refers to any kind of cellular system that can be engineered to produce a protein of the present invention. Host cells suitable for replicating and supporting the expression of the proteins of the present invention are well-known in the art. Such cells can be transfected or transduced with a particular expression vector as desired, and large numbers of cells comprising the vector can be cultivated for seeding large-scale fermenters to obtain sufficient quantities of the protein of the present invention for clinical use.

Suitable host cells include prokaryotic microorganisms such as *E.coli,* eukaryotic microorganisms such as filamentous fungi or yeast, or various eukaryotic cells such as Chinese hamster ovary Cells (CHO), insect cells, and the like. Mammalian cell lines suitable for suspension culture may be used. Examples of useful mammalian host cell lines include SV40 transformed monkey kidney CV1 line (COS-7); human embryonic kidney lines (HEK 293 or 293F cells or 293FT cells), baby hamster kidney cells (BHK), monkey kidney cells (CV1), Vero cells (VERO-76), human cervical cancer cells (HELA), canine kidney cells (MDCK), Buffalo rat liver cells (BRL 3A), human lung cells (W138), human liver cells (Hep G2), CHO cells, NSO cells, myeloma cell lines such as YO, NS0, P3X63, and Sp2/0, and the like. Mammalian host cell lines suitable for the production of proteins are known in the art. In a preferred embodiment, the host cell is a CHO or HEK293 cell or an Expi293 cell.

### IV. Production and Purification of Molecules of the Invention

In yet another aspect, the present invention provides a method for producing a hybrid protein of the present invention, the method comprising: culturing a host cell comprising a nucleic acid molecule encoding the protein under conditions suitable for expression of the hybrid protein, optionally further comprising isolating the protein from the host cell or the host cell culture medium, and/or purifying the protein.

For recombinant production, a polynucleotide encoding a protein of the present invention may be inserted into a vector for further cloning and/or expression in a host cell. Methods well known to those skilled in the art can be used to construct expression vectors. Once an expression vector comprising a polynucleotide of the present invention has been prepared for expression, the expression vector can be transfected or introduced into a suitable host cell. Various techniques can be used to achieve this purpose, for example, protoplast fusion, calcium phosphate precipitation, electroporation, retroviral transduction, viral transfection, biolistics, liposome-based transfection, or other conventional techniques.

Proteins prepared as described herein can be purified by known prior art techniques such as nickel column, high-performance liquid chromatography, affinity chromatography, ion exchange chromatography, gel electrophoresis, and the like. The actual conditions used to purify a particular protein also depend on factors such as net charge, hydrophobicity, hydrophilicity, and the like, and will be apparent to those skilled in the art.

The purity and quantification of the proteins of the present invention can be determined by any of a variety of well-known analytical methods.

### V. Assay method

The hybrid proteins provided herein can be identified, screened, or characterized for their physical/chemical properties and/or biological activity by a variety of assays known in the art.

The hemolytic inhibitory effect of the hybrid protein of the present invention can be measured by methods known in the art, such as in vitro assays and/or in vivo animal experiments. For example, a hemolytic assay, such as the method described in Examples 4.1 and 4.2, can be used to test for hemolytic inhibitory effects of the molecule on the classical and/or alternative pathways of complement immunity.

The inhibitory activity of the hybrid protein of the present invention on the complement C3b deposition can be measured by methods known in the art, such as in vitro assays and/or in vivo animal assays. For example, a hemolysis assay, such as the method described in Example 4.3, can be used to test the inhibitory effect of the molecule on the C3b deposition on the surface of red blood cells.

### VI. Pharmaceutical compositions, pharmaceutical combinations, and kits

In one aspect, the present invention provides a composition, e.g., a pharmaceutical composition, medicament, or formulation, comprising a hybrid protein of the present invention or a nucleic acid molecule encoding the same.

In one embodiment, the composition further comprises a pharmaceutical excipient, such as a pharmaceutical carrier, a pharmaceutical excipient, including buffers, as known in the art.

As used herein, "pharmaceutical carrier" includes any and all solvents, dispersion media, isotonic and absorption delaying agents, and the like that are physiologically compatible. For the use of pharmaceutical excipients and their use, see also "Handbook of Pharmaceutical Excipients", 8th Edition, R.C.Rowe, P.J.Seskey and S.C. Owen, Pharmaceutical Press, London, Chicago.

The compositions or medicaments or formulations of the present invention may be in a variety of forms. These forms include, for example, liquid, semi-solid, and solid dosage forms, such as liquid solutions (e.g., injectable solutions), powders or suspensions, liposomal formulations, and suppositories. The preferred form depends on the intended mode of administration and therapeutic use.

In addition to the hybrid protein of the present invention or the nucleic acid encoding the same, the compositions or medicaments or formulations of the present invention may also comprise additional therapeutic agents required for the particular indication being treated, preferably those that do not adversely affect the activity of each other. Thus, in one embodiment, a composition or formulation or medicament, e.g., a pharmaceutical composition, comprises a combination of one or more molecules of the present invention, and one or more additional therapeutic agents.

The compositions or medicaments or formulations of the present invention may be in a variety of forms. These forms include, for example, liquid, semi-solid, and solid dosage forms, such as liquid solutions (e.g., injectable solutions), powders or suspensions, liposomal formulations, and suppositories. The compositions or medicaments or formulations of the present invention are suitable for administration by intravenous bolus injection, intravenous infusion, intraperitoneal, intradermal, intramuscular, subcutaneous, and intranasal routes (e.g., by injection or infusion). The preferred form depends on the intended mode of administration and therapeutic use.

The present invention also provides a pharmaceutical combination or a pharmaceutical combination product comprising a hybrid protein of the present invention or a nucleic acid encoding the same and one or more other therapeutic agents.

The present invention also provides a kit of parts comprising the pharmaceutical combination, e.g., the kit of parts comprises the following in the same package:
- a first container comprising a pharmaceutical composition comprising a hybrid protein of the present invention or a nucleic acid encoding the same; and
- optionally, a second container comprising a pharmaceutical composition comprising one or more additional therapeutic agents (in some embodiments, the two or more additional therapeutic agents are in the same container, or in separate containers).

### VII. Uses and Methods

In one aspect of the present invention, provided is a method of preventing or treating a complement system-associated disease or disorder in a subject, comprising administering to the subject an effective amount of the hybrid protein or the nucleic acid encoding the same of the present invention or the composition or medicament or formulation comprising the protein.

In some embodiments, the complement system-associated disease is due to abnormal activation of the complement system or a dysregulation of the complement system. In some embodiments, the abnormal activation of the complement system or dysregulation of the complement system is due to, for example, microbial infection or an increase in autoimmune antibodies, or to a decrease, deletion, incapacitation, or interference or blockade of a function of complement regulatory proteins, etc.

In some embodiments, the disease treatment will benefit from inhibiting the activity of the complement system.

In other aspects, the present invention provides the use of the molecule of the present invention, or a composition comprising the same, in the manufacture or preparation of a medicament for use as described herein, for example, for the prevention or treatment of a complement system-associated disease or disorder as mentioned herein.

In some embodiments, the complement system-associated disease or disorder can be a disease requiring hemolysis inhibition, e.g., a disease requiring inhibition of hemolysis of the classical pathway of the complement immunity and/or the alternative pathway of the complement immunity.

In some embodiments, the complement system-associated disease or disorder is a disease in which inhibition of C3b deposition activity is desired, such as Dense Deposit Disease (DDD).

In some embodiments, the hybrid protein or the nucleic acid encoding the same of the present invention or the composition or medicament or formulation comprising the proteins or nucleic acids delays the onset of a disorder and/or a symptom associated with the disorder.

In some embodiments, the hybrid protein or the nucleic acid encoding the same of the present invention or the composition or medicament or formulation comprising the proteins or nucleic acids can also be administered in combination with one or more other therapies, e.g., therapeutic modalities and/or other therapeutic agents, for use as described herein, e.g., for the prevention and/or treatment of the related diseases or disorders as mentioned herein.

The routes of administration of the hybrid protein or the nucleic acid encoding the same of the present invention or the composition or medicament or formulation comprising the proteins or nucleic acids depend on known methods, e.g., injection or infusion.

The invention further encompasses the use of the hybrid protein or the nucleic acid encoding the same of the present invention or the composition or formulation comprising the proteins or nucleic acids in the manufacture of a medicament for use as described herein, e.g., for the prevention and/or treatment of a related disease or disorder as mentioned herein.

### Examples

### Example 1. Molecular construction

In order to construct the protein expression vector of the hybrid molecule involved in the present invention and the complement regulatory molecule of the prior art, the DNA encoding each protein was subcloned into the pcDNA3.1 expression vector (purchased from Biofeng) with the synthesized gene DNA (GENEWIZ Inc., Suzhou/Sangon Biotech (Shanghai) Co., Ltd.) as a template by using a method of conventional molecular cloning technology. The plasmid was verified by sequencing and then was used for transient protein expression. Each encoded protein was added with a signal peptide MGWSCIILFLVATATGVHS to the N-terminus and a GHHHHHH 6×His tag to the C-terminus to facilitate subsequent secretion expression with mammalian cells and nickel column purification.

The amino acid sequence of each protein is as follows:
>CR13m (human CR1 CCP1-3 or SCR1-3, comprising N29K, S37Y, G79D and D109N mutations, the sequence is derived from patent US9988611_B2): SEQ ID NO: 34 
QCNAPEWLPFARPTNLTDEFEFPIGTYLKYECRPGYYGRPFSIICLKNSVWTGA
KDRCRRKSCRNPPDPVNGMVHVIKDIQFGSQIKYSCTKGYRLIGSSSATCIISG
NTVIWDNETPICDRIPCGLPPTITNGDFISTNRENFHYGSVVTYRCNPGSGGRK
VFELVGEPSIYCTSNDDQVGIWSGPAPQCIGHHHHHH
>D24 (human DAF/CD55 CCP2-4 or SCR2-4, the sequence can take reference from Hui-fen Zhang et al., THE JOURNAL OF BIOLOGICAL CHEMISTRY, Vol. 276, No. 29, Issue of July 20, pp. 27290-27295, 2001): SEQ ID NO: 35 
SCEVPTRLNSASLKQPYITQNYFPVGTVVEYECRPGYRREPSLSPKLTCLQNLK
WSTAVEFCKKKSCPNPGEIRNGQIDVPGGILFGATISFSCNTGYKLFGSTSS
FCLISGSSVQWSDPLPECREIYCPAPPQIDNGIIQGERDHYGYRQSVTYAC
NKGFTMIGEHSIYCTVNNDEGEWSGPPPECRGGHHHHHH
>F15 (human CFH CCP1-5 or SCR1-5, comprising a V62I mutation, the sequence can take reference from Masha Fridkis-Hareli et al., Blood. 2011 Oct 27; 118(17): 4705-4713 and Agustín Tortajada et al., Hum Mol Genet. 2009 September 15; 18(18):3452-3461): SEQ ID NO: 36 
EDCNELPPRRNTEILTGSWSDQTYPEGTQAIYKCRPGYRSLGNIIMVCRKGEW
VALNPLRKCQKRPCGHPGDTPFGTFTLTGGNVFEYGVKAVYTCNEGYQLLGE
INYRECDTDGWTNDIPICEVVKCLPVTAPENGKIVSSAMEPDREYHFGQAVRF
VCNSGYKIEGDEEMHCSDDGFWSKEKPKCVEISCKSPDVINGSPISQKIIYKEN
ERFQYKCNMGYEYSERGDAVCTESGWRPLPSCEEKSCDNPYIPNGDYSPLRIK
HRTGDEITYQCRNGFYPATRGNTAKCTSTGWIPAPRCTLKPGHHHHHH
>F1D34-1 (hybrid molecule 1 of the present invention, human CFH CCP1 or SCR1 - human DAF/CD55 CCP3-4 or SCR3-4): SEQ ID NO: 22, the sequence with a signal peptide connected to the N terminus is SEQ ID NO: 26
>F1D34-2i (hybrid molecule 2 of the present invention, human CFH CCP1 or SCR1 - human DAF/CD55 CCP3-4 or SCR3-4, comprising a V62I mutation): SEQ ID NO: 24, the amino acid sequence with a signal peptide sequence connected to the N terminus is SEQ ID NO: 28
>CR15D34 (hybrid molecule 3 of the present invention, human CR1 CCP15 or SCR15 - human DAF/CD55 CCP3-4 or SCR3-4): SEQ ID NO: 37 
GHCQAPDHFLFAKLKTQTNASDFPIGTSLKYECRPEYYGRPFSITCLDNLVWS
SPKDVCKRKSCPNPGEIRNGQIDVPGGILFGATISFSCNTGYKLFGSTSSFC
LISGSSVQWSDPLPECREIYCPAPPQIDNGIIQGERDHYGYRQSVTYACNK
GFTMIGEHSIYCTVNNDEGEWSGPPPECRGGHHHHHH
>FH13 (human CFH CCP1-3 or SCR1-3, comprising a V62I mutation, there are two more amino acids at the C-terminus for connecting His purification tag): SEQ ID NO: 38 
EDCNELPPRRNTEILTGSWSDQTYPEGTQAIYKCRPGYRSLGNIIMVCRKGEW
VALNPLRKCQKRPCGHPGDTPFGTFTLTGGNVFEYGVKAVYTCNEGYQLLGE
INYRECDTDGWTNDIPICEVVKCLPVTAPENGKIVSSAMEPDREYHFGQAVRF
VCNSGYKIEGDEEMHCSDDGFWSKEKPKCVEISGHHHHHH
>D14 (human DAF/CD55 CCP1-4 or SCR1-4, no leader sequence, there are two more amino acids at the C-terminus for connecting His purification tag): SEQ ID NO: 39

DCGLPPDVPNAQPALEGRTSFPEDTVITYKCEESFVKIPGEKDSVICLKGSQWS
DIEEFCNRSCEVPTRLNSASLKQPYITQNYFPVGTVVEYECRPGYRREPSLSPK
LTCLQNLKWSTAVEFCKKKSCPNPGEIRNGQIDVPGGILFGATISFSCNTGYKL
FGSTSSFCLISGSSVQWSDPLPECREIYCPAPPQIDNGIIQGERDHYGYRQSVTY
ACNKGFTMIGEHSIYCTVNNDEGEWSGPPPECRGKSGHHHHHH

### Example 2. Protein expression

After amplification of the plasmid with strain DH5 alpha (Yeasen Biotechnology), the plasmid was prepared using the NucleoBond Xtra Midi Plus (MACHEREY-NAGEL, see the product Manual for details). Expi293 cells (purchased from Thermo fisher) were transfected with the prepared plasmids using PEI (Polyscience) for transient expression of proteins (see Jäger, V., et al., BMC Biotechnol 13, 52, 2013. for the transfection method). The harvested supernatant was purified by affinity chromatography in a nickel ion pre-packed column (GE life sciences, HisTrap HP, see product Manual for details), dialyzed against PBS for fluid changes, the fluid change protein samples were sterilized by filtration through 0.2 µm filters, and protein quantification was performed according to the theoretical extinction coefficient using the NanoDrop (Thermofisiher) A280 method.

### Example 3. Protein purity detection (SDS-PAGE)

2.5 µg of protein was each taken and added proportionally to the loading buffer (Sangon Biotech) with reducing agent and without reducing agent, respectively. The samples with loading buffer containing reducing agent were boiled at 95°C for 5 minutes to fully denature the proteins. For the samples with loading buffer without reducing agent, no heat treatment was performed. Electrophoresis was performed with a 10% Precast-GLgel Tris-Glycine precast gel (Sangon Biotech) in a Mini-PROTEAN^{®} Tetra electrophoresis system (BioRad). After completing the electrophoresis, the gel was stained with 0.1% Coomassie Brilliant Blue staining solution and de-stained with an ethanol-glacial acetic acid solution.

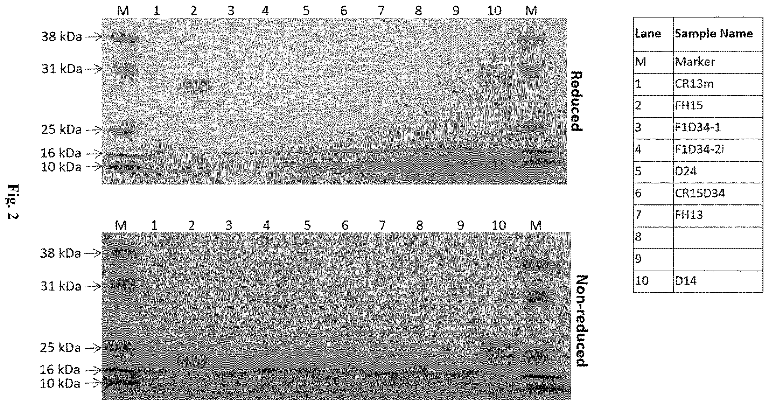

The SDS-PAGE electrophoresis results showed that except for a slight diffusion of the D14 protein band, the other protein bands were relatively clear, indicating that the purity of the proteins under both reducing and non-reducing conditions was good (as shown in Figure 2). Additionally, due to differences in protein reduction, denaturation, modification status, and electrophoresis conditions, there was a certain discrepancy between the electrophoretic molecular weight and the theoretical molecular weight.

### Example 4. Detection of complement-inhibiting activity

### 4.1 Detection of inhibitory activity on Classical Pathway of complement (CP)

In this experiment, the degree of inhibition of hemolysin-mediated hemolysis of sheep red blood cells caused by normal human serum complement activity was measured by testing the protein to be tested at different concentrations, and the inhibitory activity of each protein on complement CP was analyzed and compared.

The experimental process was briefly described as follows:
(1) Gradient dilution of proteins: the protein was formulated into a 12000 nM solution with GVB++ buffer (Gelatin Flora Buffer comprising Ca²⁺ and Mg²⁺, Cat. No.: 25-02080, TIANDZ), followed by 4-fold gradient dilution, with a total of 7 gradients;
(2) Dilution of serum: The normal human serum NHS (Shanghai Schbio Co., Ltd.) was taken out of the -70°C refrigerator, followed by naturally thawing at 4°C and then the serum was diluted to 4% with GVB++ buffer;
(3) Activation of sheep red blood cells: the sheep red blood cells (Nanjing SenBeiJia Biological Technology Co., Ltd.) were washed with GVB++ buffer until the supernatant was transparent, then resuspended with GVB++ buffer, and hemolysin (BM351Y, Beijing Bersee Technology Co., Ltd.) was added to the red blood cell suspension at a ratio of 1:400, followed by incubation at 4°C for 15 minutes to activate the sheep red blood cells. The activated sheep red blood cells were washed twice with GVB++ and resuspended in GVB++ buffer;
(4) Incubation: 25 µL each of the diluted protein and serum were mixed in a 96-well plate, then 50 µL of the red blood cell suspension obtained in step (3) was added, resulting in an initial final concentration of the protein of 3000 nM and a final concentration of the serum of 1%. The mixture was blended thoroughly and placed in a 37°C constant temperature incubator for 1 hour, with a negative control (comprising only serum) and a positive control (comprising only serum and red blood cells);
(5) Termination: After the incubation, 100 µL of 20 mM EDTA-GVB buffer (GVBE) was added to each reaction well to terminate the reaction;
(6) Reading value: The 96-well plate was centrifuged at 3000 rpm for 5 minutes. 100 µL of the supernatant was added into a new 96-well flat-bottom plate. The absorbance value at OD405nm was measured in a multi-function plate reader, and the data were saved;
(7) Data processing: the obtained OD405nm reading value was put into the following formula to calculate the red blood cell hemolysis inhibition rate: Hemolysis inhibition rate (%) = (positive control reading value - experimental group reading value) / (positive control reading value - negative control reading value) * 100%

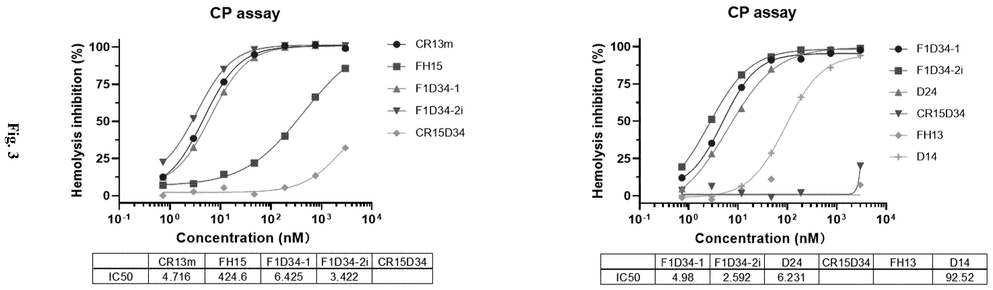

Then the IC50 value of the protein was calculated by plotting a four-parameter fit curve, with the final protein concentration as the abscissa and the hemolysis inhibition rate as the ordinate model. The results were shown in Figure 3.

The experimental test results of CP hemolytic inhibitory activity showed that the hybrid proteins of CFH and DAF F1D34-1 and F1D34-2i both had strong CP inhibitory activity, which was equivalent to that of CR1 activity-enhancing fragment CR13m and DAF fragment D24, significantly better than that of CFH fragment FH15 and DAF fragment D14, and also far superior to that of CR15D34, a hybrid protein of CR1 and DAF. Only weak CP inhibitory activity was detected for CR15D34, while CFH fragment FH13 had little activity (as shown in Figure 3), indicating that the hybrid protein obtained through domain hybridization had excellent CP inhibitory activity.

### 4.2 Detection of inhibitory activity on Alternative Pathway of complement (AP)

In this experiment, the degree of inhibition of rabbit erythrocyte hemolysis caused by normal human serum complement activity was measured by testing the protein to be tested at different concentrations, and the inhibitory activity of each protein on complement AP was analyzed and compared.

The experimental process was briefly described as follows:
(1) Gradient dilution of proteins: The protein was formulated into a 28000nM solution with GVBMG buffer (Gelatin Flora Buffer comprising Mg²⁺ and EGTA, Cat. No.: 25-02090, TIANDZ), followed by 4-fold gradient dilution, with a total of 7 gradients;
(2) Dilution of serum: The normal human serum NHS (Shanghai Schbio Co., Ltd.) was taken out of the -70°C refrigerator, followed by naturally thawing at 4°C and the serum was diluted to 60% with GVBMG;
(3) Preparation of rabbit red blood cells: rabbit red blood cells (Nanjing SenBeiJia Biological Technology Co., Ltd.) were washed with GVBMG until the supernatant was transparent, and then resuspended with GVBMG;
(4) Incubation: 25 µL each of the diluted protein and serum was mixed in a 96-well plate, then 50 µL of the red blood cell suspension obtained in step (3) was added, resulting in an initial final concentration of the protein of 7000 nM and a final concentration of the serum of 15%, the mixture was blended thoroughly and placed in a 37°C constant temperature incubator for 1 hour, with a negative control (comprising only serum) and a positive control (comprising only serum and red blood cells);
(5) Termination: After the incubation, 100 µL of 10 mM EDTA-GVB buffer (GVBE) was added to each reaction well to terminate the reaction;
(6) Reading value: The 96-well plate was centrifuged at 3000 rpm for 5 minutes. 100 µL of the supernatant was added into a new 96-well flat-bottom plate. The absorbance value at OD405nm was measured in a multi-function plate reader, and the data were saved;
(7) Data processing: The obtained OD405nm reading value was put into the following formula to calculate the red blood cell hemolysis inhibition rate: Hemolysis inhibition rate (%) = (positive control reading value - experimental group reading value) / (positive control reading value - negative control reading value) * 100%

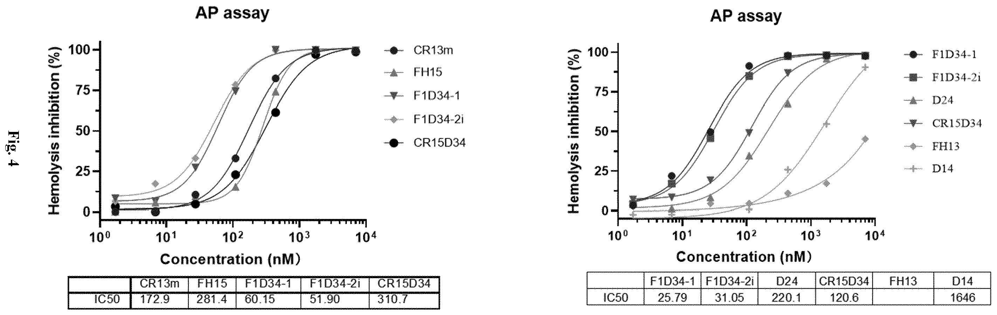

Then the IC50 value of the antibody was calculated by plotting a four-parameter fit curve, with the final protein concentration as the abscissa and the hemolysis inhibition rate as the ordinate. The results were shown in Figure 4.

The experimental test results of AP hemolytic inhibitory activity showed that the hybrid proteins of CFH and DAF F1D34-1 and F1D34-2i both had strong AP inhibitory activity, which was better than that of CFH fragments FH15 and FH13, and DAF fragments D24 and D14, and was also superior to that of the hybrid protein of CR1 and DAF CR15D34 and the CR1 activity-enhancing fragment CR13m (as shown in Figure 4), indicating that the hybrid protein obtained through domain hybridization had more excellent AP inhibitory activity.

### 4.3 Detection of inhibitory activity in complement C3b deposition

In this experiment, the inhibition of C3b deposition on the surface of rabbit red blood cells by different complement regulatory proteins after human complement activation was detected, and the inhibitory activity of each protein on C3b deposition was analyzed and compared.

The experimental process was briefly described as follows:
(1) Gradient dilution of proteins: The protein was prepared into a 1400 nM solution with GVBMG buffer (Gelatin Flora Buffer comprising Mg²⁺ and EGTA, Cat. No.: 25-02090, TIANDZ), followed by 3-fold gradient dilution, with a total of 7 gradients;
(2) Dilution of serum: In order to prevent complement activation from causing hemolysis and rupture of rabbit red blood cells, resulting in the inability to accurately detect the deposition of C3b on the cell surface, the C5-depleted normal human serum was used for the experiment. C5-Dpl NHS (Complement Technology) was taken out of the -70°C refrigerator, followed by naturally thawing at 4°C and the serum was diluted to 40% with GVBMG;
(3) Preparation of rabbit red blood cells: 4% rabbit red blood cells (Nanjing SenBeiJia Biological Technology Co., Ltd.) were washed with GVBMG until the supernatant was transparent, and then resuspended with GVBMG to a cell density of 1×10⁷ cells/mL;
(4) Incubation: 25 µL each of the diluted protein and serum were mixed in a 96-well plate, then 50 µL of the red blood cell suspension obtained in step (3) was added, resulting in an initial final concentration of the protein of 350 nM and a final concentration of the serum of 10%, the mixture was blended thoroughly and placed in a 37°C constant-temperature incubator for 1 hour, with a negative control (comprising only complement-inactivated serum and red blood cells) and a positive control (comprising only normal serum and red blood cells);
(5) Termination: After the incubation, 100 µL of 10 mM EDTA-GVB buffer (GVBE) was added to each reaction well to terminate the reaction;
(6) Detection: the incubated cells were washed three times with PBS, fluorescein-labeled anti-human C3b/iC3b antibody was added for staining (APC anti-complement C3b/iC3b Antibody, Biolegend), followed by incubation at 4°C for 30 minutes and washing again 3 times. The APC fluorescence MFI (mean fluorescence intensity) of each sample was measured with a flow cytometer (Cytoflex, Beckman), and the data were saved;
(7) Data processing: the deposition rate of C3b on the surface of red blood cells was calculated with the following formula: Deposition rate (%) = experimental group MFI value / positive control MFI value * 100%

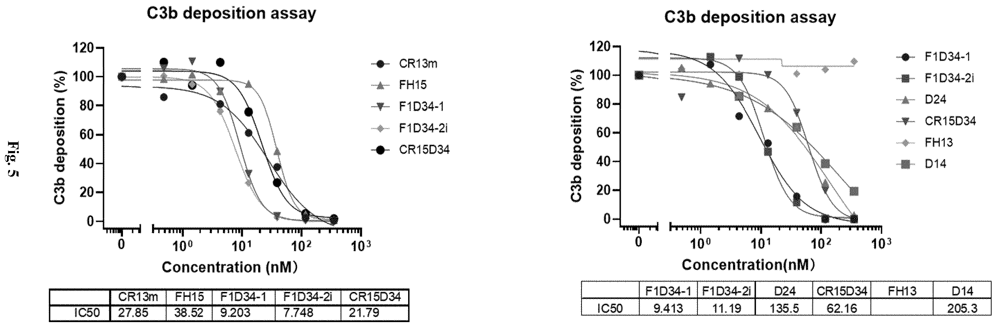

Then the IC50 value of the antibody was calculated by plotting a four-parameter fit curve, with the final protein concentration as the abscissa and the deposition rate as the ordinate. The results were shown in Figure 5.

The experimental test results of C3b deposition showed that the hybrid proteins of CFH and DAF F1D34-1 and F1D34-2i both had strong C3b deposition inhibitory activity, which was better than that of the CFH fragments FH15 and FH13 and the DAF fragments D24 and D14, and was also better than that of the hybrid protein of CR1 and DAF CR15D34 and the CR1 activity-enhancing fragment CR13m (as shown in Figure 5), indicating that the hybrid protein obtained through domain hybridization had more excellent C3b deposition inhibitory activity.

### Sequence listing

| | |
|---|---|
| SEQ ID NO:1 | MTVARPSVPAALPLLGELPRLLLLVLLCLPAVWGDCGLPPDVPNAQPALEGRTSFPEDTVITYKCEESFVKIPGEKDSV ICLKGSQWSDIEEFCNRSCEVPTRLNSASLKQPYITQNYFPVGTVVEYECRPGYRREPSLSPKLTCLQNLKWSTAVEFC KKKSCPNPGEIRNGQIDVPGGILFGATISFSCNTGYKLFGSTSSFCLISGSSVQWSDPLPECREIYCPAPPQIDNGIIQG ERDHYGYRQSVTYACNKGFTMIGEHSIYCTVNNDEGEWSGPPPECRGKSLTSKVPPTVQKPTTVNVPTTEVSPTSQ KTTTKTTTPNAQATRSTPVSRTTKHFHETTPNKGSGTTSGTTRLLSGHTCFTLTGLLGTLVTMGLLT |
| SEQ ID NO:2 | ATGACCGTCGCGCGGCCGAGCGTGCCCGCGGCGCTGCCCCTCCTCGGGGAGCTGCCCCGGCTGCTGCTGCTG GTGCTGTTGTGCCTGCCGGCCGTGTGGGGTGACTGTGGCCTTCCCCCAGATGTACCTAATGCCCAGCCAGCTTT GGAAGGCCGTACAAGTTTTCCCGAGGATACTGTAATAACGTACAAATGTGAAGAAAGCTTTGTGAAAATTCCTG GCGAGAAGGACTCAGTGATCTGCCTTAAGGGCAGTCAATGGTCAGATATTGAAGAGTTCTGCAATCGTAGCTGC GAGGTGCCAACAAGGCTAAATTCTGCATCCCTCAAACAGCCTTATATCACTCAGAATTATTTTCCAGTCGGTACTG TTGTGGAATATGAGTGCCGTCCAGGTTACAGAAGAGAACCTTCTCTATCACCAAAACTAACTTGCCTTCAGAATT TAAAATGGTCCACAGCAGTCGAATTTTGTAAAAAGAAATCATGCCCTAATCCGGGAGAAATACGAAATGGTCAG ATTGATGTACCAGGTGGCATATTATTTGGTGCAACCATCTCCTTCTCATGTAACACAGGGTACAAATTATTTGGCTC GACTTCTAGTTTTTGTCTTATTTCAGGCAGCTCTGTCCAGTGGAGTGACCCGTTGCCAGAGTGCAGAGAAATTTA TTGTCCAGCACCACCACAAATTGACAATGGAATAATTCAAGGGGAACGTGACCATTATGGATATAGACAGTCTGT AACGTATGCATGTAATAAAGGATTCACCATGATTGGAGAGCACTCTATTTATTGTACTGTGAATAATGATGAAGGA GAGTGGAGTGGCCCACCACCTGAATGCAGAGGAAAATCTCTAACTTCCAAGGTCCCACCAACAGTTCAGAAAC CTACCACAGTAAATGTTCCAACTACAGAAGTCTCACCAACTTCTCAGAAAACCACCACAAAAACCACCACACCA AATGCTCAAGCAACACGGAGTACACCTGTTTCCAGGACAACCAAGCATTTTCATGAAACAACCCCAAATAAAGG AAGTGGAACCACTTCAGGTACTACCCGTCTTCTATCTGGGCACACGTGTTTCACGTTGACAGGTTTGCTTGGGA CGCTAGTAACCATGGGCTTGCTGACTTAG |
| SEQ ID NO:3 | MRLLAKIICLMLWAICVAEDCNELPPRRNTEILTGSWSDQTYPEGTQAIYKCRPGYRSLGNVIMVCRKGEWVALNPL RKCQKRPCGHPGDTPFGTFTLTGGNVFEYGVKAVYTCNEGYQLLGEINYRECDTDGWTNDIPICEVVKCLPVTAPE NGKIVSSAMEPDREYHFGQAVRFVCNSGYKIEGDEEMHCSDDGFWSKEKPKCVEISCKSPDVINGSPISQKIIYKEN ERFQYKCNMGYEYSERGDAVCTESGWRPLPSCEEKSCDNPYIPNGDYSPLRIKHRTGDEITYQCRNGFYPATRGNTA KCTSTGWIPAPRCTLKPCDYPDIKHGGLYHENMRRPYFPVAVGKYYSYYCDEHFETPSGSYWDHIHCTQDGWSPAV PCLRKCYFPYLENGYNQNYGRKFVQGKSIDVACHPGYALPKAQTTVTCMENGWSPTPRCIRVKTCSKSSIDIENGFI SESQYTYALKEKAKYQCKLGYVTADGETSGSITCGKDGWSAQPTCIKSCDIPVFMNARTKNDFTWFKLNDTLDYEC HDGYESNTGSTTGSIVCGYNGWSDLPICYERECELPKIDVHLVPDRKKDQYKVGEVLKFSCKPGFTIVGPNSVQCYH FGLSPDLPICKEQVQSCGPPPELLNGNVKEKTKEEYGHSEVVEYYCNPRFLMKGPNKIQCVDGEWTTLPVCIVEEST CGDIPELEHGWAQLSSPPYYYGDSVEFNCSESFTMIGHRSITCIHGVWTQLPQCVAIDKLKKCKSSNLIILEEHLKNKK EFDHNSNIRYRCRGKEGWIHTVCINGRWDPEVNCSMAQIQLCPPPPQIPNSHNMTTTLNYRDGEKVSVLCQENY LIQEGEEITCKDGRWQSIPLCVEKIPCSQPPQIEHGTINSSRSSQESYAHGTKLSYTCEGGFRISEENETTCYMGKWSS PPQCEGLPCKSPPEISHGVVAHMSDSYQYGEEVTYKCFEGFGIDGPAIAKCLGEKWSHPPSCIKTDCLSLPSFENAIP MGEKKDVYKAGEQVTYTCATYYKMDGASNVTCINSRWTGRPTCRDTSCVNPPTVQNAYIVSRQMSKYPSGERVR YQCRSPYEMFGDEEVMCLNGNWTEPPQCKDSTGKCGPPPPIDNGDITSFPLSVYAPASSVEYQCQNLYQLEGNKRI TCRNGQWSEPPKCLHPCVISREIMENYNIALRWTAKQKLYSRTGESVEFVCKRGYRLSSRSHTLRTTCWDGKLEYPT CAKR |
| SEQ ID NO:4 | ATGAGACTTCTAGCAAAGATTATTTGCCTTATGTTATGGGCTATTTGTGTAGCAGAAGATTGCAATGAACTTCCTC CAAGAAGAAATACAGAAATTCTGACAGGTTCCTGGTCTGACCAAACATATCCAGAAGGCACCCAGGCTATCTAT AAATGCCGCCCTGGATATAGATCTCTTGGAAATGTAATAATGGTATGCAGGAAGGGAGAATGGGTTGCTCTTAAT CCATTAAGGAAATGTCAGAAAAGGCCCTGTGGACATCCTGGAGATACTCCTTTTGGTACTTTTACCCTTACAGGA GGAAATGTGTTTGAATATGGTGTAAAAGCTGTGTATACATGTAATGAGGGGTATCAATTGCTAGGTGAGATTAATT ACCGTGAATGTGACACAGATGGATGGACCAATGATATTCCTATATGTGAAGTTGTGAAGTGTTTACCAGTGACAG CACCAGAGAATGGAAAAATTGTCAGTAGTGCAATGGAACCAGATCGGGAATACCATTTTGGACAAGCAGTACG GTTTGTATGTAACTCAGGCTACAAGATTGAAGGAGATGAAGAAATGCATTGTTCAGACGATGGTTTTTGGAGTA AAGAGAAACCAAAGTGTGTGGAAATTTCATGCAAATCCCCAGATGTTATAAATGGATCTCCTATATCTCAGAAGA TTATTTATAAGGAGAATGAACGATTTCAATATAAATGTAACATGGGTTATGAATACAGTGAAAGAGGAGATGCTGT ATGCACTGAATCTGGATGGCGTCCGTTGCCTTCATGTGAAGAAAAATCATGTGATAATCCTTATATTCCAAATGGT GACTACTCACCTTTAAGGATTAAACACAGAACTGGAGATGAAATCACGTACCAGTGTAGAAATGGTTTTTATCCT GCAACCCGGGGAAATACAGCAAAATGCACAAGTACTGGCTGGATACCTGCTCCGAGATGTACCTTGAAACCTTG TGATTATCCAGACATTAAACATGGAGGTCTATATCATGAGAATATGCGTAGACCATACTTTCCAGTAGCTGTAGGA AAATATTACTCCTATTACTGTGATGAACATTTTGAGACTCCGTCAGGAAGTTACTGGGATCACATTCATTGCACAC AAGATGGATGGTCGCCAGCAGTACCATGCCTCAGAAAATGTTATTTTCCTTATTTGGAAAATGGATATAATCAAAA TCATGGAAGAAAGTTTGTACAGGGTAAATCTATAGACGTTGCCTGCCATCCTGGCTACGCTCTTCCAAAAGCGCA GACCACAGTTACATGTATGGAGAATGGCTGGTCTCCTACTCCCAGATGCATCCGTGTCAAAACATGTTCCAAATC AAGTATAGATATTGAGAATGGGTTTATTTCTGAATCTCAGTATACATATGCCTTAAAAGAAAAAGCGAAATATCAA TGCAAACTAGGATATGTAACAGCAGATGGTGAAACATCAGGATCAATTACATGTGGGAAAGATGGATGGTCAGC TCAACCCACGTGCATTAAATCTTGTGATATCCCAGTATTTATGAATGCCAGAACTAAAAATGACTTCACATGGTTTA AGCTGAATGACACATTGGACTATGAATGCCATGATGGTTATGAAAGCAATACTGGAAGCACCACTGGTTCCATAG TGTGTGGTTACAATGGTTGGTCTGATTTACCCATATGTTATGAAAGAGAATGCGAACTTCCTAAAATAGATGTACA |
| | CTTAGTTCCTGATCGCAAGAAAGACCAGTATAAAGTTGGAGAGGTGTTGAAATTCTCCTGCAAACCAGGATTTA CAATAGTTGGACCTAATTCCGTTCAGTGCTACCACTTTGGATTGTCTCCTGACCTCCCAATATGTAAAGAGCAAGT ACAATCATGTGGTCCACCTCCTGAACTCCTCAATGGGAATGTTAAGGAAAAAACGAAAGAAGAATATGGACACA GTGAAGTGGTGGAATATTATTGCAATCCTAGATTTCTAATGAAGGGACCTAATAAAATTCAATGTGTTGATGGAG AGTGGACAACTTTACCAGTGTGTATTGTGGAGGAGAGTACCTGTGGAGATATACCTGAACTTGAACATGGCTGG GCCCAGCTTTCTTCCCCTCCTTATTACTATGGAGATTCAGTGGAATTCAATTGCTCAGAATCATTTACAATGATTGG ACACAGATCAATTACGTGTATTCATGGAGTATGGACCCAACTTCCCCAGTGTGTGGCAATAGATAAACTTAAGAA GTGCAAATCATCAAATTTAATTATACTTGAGGAACATTTAAAAAACAAGAAGGAATTCGATCATAATTCTAACATA AGGTACAGATGTAGAGGAAAAGAAGGATGGATACACACAGTCTGCATAAATGGAAGATGGGATCCAGAAGTGA ACTGCTCAATGGCACAAATACAATTATGCCCACCTCCACCTCAGATTCCCAATTCTCACAATATGACAACCACACT GAATTATCGGGATGGAGAAAAAGTATCTGTTCTTTGCCAAGAAAATTATCTAATTCAGGAAGGAGAAGAAATTA CATGCAAAGATGGAAGATGGCAGTCAATACCACTCTGTGTTGAAAAAATTCCATGTTCACAACCACCTCAGATAG AACACGGAACCATTAATTCATCCAGGTCTTCACAAGAAAGTTATGCACATGGGACTAAATTGAGTTATACTTGTG AGGGTGGTTTCAGGATATCTGAAGAAAATGAAACAACATGCTACATGGGAAAATGGAGTTCTCCACCTCAGTGT GAAGGCCTTCCTTGTAAATCTCCACCTGAGATTTCTCATGGTGTTGTAGCTCACATGTCAGACAGTTATCAGTATG GAGAAGAAGTTACGTACAAATGTTTTGAAGGTTTTGGAATTGATGGGCCTGCAATTGCAAAATGCTTAGGAGA AAAATGGTCTCACCCTCCATCATGCATAAAAACAGATTGTCTCAGTTTACCTAGCTTTGAAAATGCCATACCCATG GGAGAGAAGAAGGATGTGTATAAGGCGGGTGAGCAAGTGACTTACACTTGTGCAACATATTACAAAATGGATG GAGCCAGTAATGTAACATGCATTAATAGCAGATGGACAGGAAGGCCAACATGCAGAGACACCTCCTGTGTGAAT CCGCCCACAGTACAAAATGCTTATATAGTGTCGAGACAGATGAGTAAATATCCATCTGGTGAGAGAGTACGTTAT CAATGTAGGAGCCCTTATGAAATGTTTGGGGATGAAGAAGTGATGTGTTTAAATGGAAACTGGACGGAACCAC CTCAATGCAAAGATTCTACAGGAAAATGTGGGCCCCCTCCACCTATTGACAATGGGGACATTACTTCATTCCCGT TGTCAGTATATGCTCCAGCTTCATCAGTTGAGTACCAATGCCAGAACTTGTATCAACTTGAGGGTAACAAGCGAA TAACATGTAGAAATGGACAATGGTCAGAACCACCAAAATGCTTACATCCGTGTGTAATATCCCGAGAAATTATGG AAAATTATAACATAGCATTAAGGTGGACAGCCAAACAGAAGCTTTATTCGAGAACAGGTGAATCAGTTGAATTT GTGTGTAAACGGGGATATCGTCTTTCATCACGTTCTCACACATTGCGAACAACATGTTGGGATGGGAAACTGGA GTATCCAACTTGTGCAAAAAGATAG |
| SEQ ID NO:5 | MRLLAKIICLMLWAICVAEDCNELPPRRNTEILTGSWSDQTYPEGTQAIYKCRPGYRSLGNVIMVCRKGEWVALNPL RKCQKRPCGHPGDTPFGTFTLTGGNVFEYGVKAVYTCNEGYQLLGEINYRECDTDGWTNDIPICEVVKCLPVTAPE NGKIVSSAMEPDREYHFGQAVRFVCNSGYKIEGDEEMHCSDDGFWSKEKPKCVEISCKSPDVINGSPISQKIIYKEN ERFQYKCNMGYEYSERGDAVCTESGWRPLPSCEEKSCDNPYIPNGDYSPLRIKHRTGDEITYQCRNGFYPATRGNTA KCTSTGWIPAPRCTLKPCDYPDIKHGGLYHENMRRPYFPVAVGKYYSYYCDEHFETPSGSYWDHIHCTQDGWSPAV PCLRKCYFPYLENGYNQNYGRKFVQGKSIDVACHPGYALPKAQTTVTCMENGWSPTPRCIRVSFTL |
| SEQ ID NO:6 | ATGAGACTTCTAGCAAAGATTATTTGCCTTATGTTATGGGCTATTTGTGTAGCAGAAGATTGCAATGAACTTCCTC CAAGAAGAAATACAGAAATTCTGACAGGTTCCTGGTCTGACCAAACATATCCAGAAGGCACCCAGGCTATCTAT AAATGCCGCCCTGGATATAGATCTCTTGGAAATGTAATAATGGTATGCAGGAAGGGAGAATGGGTTGCTCTTAAT CCATTAAGGAAATGTCAGAAAAGGCCCTGTGGACATCCTGGAGATACTCCTTTTGGTACTTTTACCCTTACAGGA GGAAATGTGTTTGAATATGGTGTAAAAGCTGTGTATACATGTAATGAGGGGTATCAATTGCTAGGTGAGATTAATT ACCGTGAATGTGACACAGATGGATGGACCAATGATATTCCTATATGTGAAGTTGTGAAGTGTTTACCAGTGACAG CACCAGAGAATGGAAAAATTGTCAGTAGTGCAATGGAACCAGATCGGGAATACCATTTTGGACAAGCAGTACG GTTTGTATGTAACTCAGGCTACAAGATTGAAGGAGATGAAGAAATGCATTGTTCAGACGATGGTTTTTGGAGTA AAGAGAAACCAAAGTGTGTGGAAATTTCATGCAAATCCCCAGATGTTATAAATGGATCTCCTATATCTCAGAAGA TTATTTATAAGGAGAATGAACGATTTCAATATAAATGTAACATGGGTTATGAATACAGTGAAAGAGGAGATGCTGT ATGCACTGAATCTGGATGGCGTCCGTTGCCTTCATGTGAAGAAAAATCATGTGATAATCCTTATATTCCAAATGGT GACTACTCACCTTTAAGGATTAAACACAGAACTGGAGATGAAATCACGTACCAGTGTAGAAATGGTTTTTATCCT GCAACCCGGGGAAATACAGCAAAATGCACAAGTACTGGCTGGATACCTGCTCCGAGATGTACCTTGAAACCTTG TGATTATCCAGACATTAAACATGGAGGTCTATATCATGAGAATATGCGTAGACCATACTTTCCAGTAGCTGTAGGA AAATATTACTCCTATTACTGTGATGAACATTTTGAGACTCCGTCAGGAAGTTACTGGGATCACATTCATTGCACAC AAGATGGATGGTCGCCAGCAGTACCATGCCTCAGAAAATGTTATTTTCCTTATTTGGAAAATGGATATAATCAAAA TCATGGAAGAAAGTTTGTACAGGGTAAATCTATAGACGTTGCCTGCCATCCTGGCTACGCTCTTCCAAAAGCGCA GACCACAGTTACATGTATGGAGAATGGCTGGTCTCCTACTCCCAGATGCATCCGTGTCAGCTTTACCCTCTGA |
| SEQ ID NO:7 | KSCPNPGEIRNGQIDVPGGILFGATISFSCNTGYKLFGSTSSFCLISGSSVQWSDPLPECRE |
| SEQ ID NO:8 | CPNPGEIRNGQIDVPGGILFGATISFSCNTGYKLFGSTSSFCLISGSSVQWSDPLPECRE |
| SEQ ID NO:9 | IYCPAPPQIDNGIIQGERDHYGYRQSVTYACNKGFTMIGEHSIYCTVNNDEGEWSGPPPECRG |
| SEQ ID NO:10 | KSCPNPGEIRNGQIDVPGGILFGATISFSCNTGYKLFGSTSSFCLISGSSVQWSDPLPECREIYCPAPPQIDNGIIQGER DHYGYRQSVTYACNKGFTMIGEHSIYCTVNNDEGEWSGPPPECRG |
| SEQ ID NO:11 | CPNPGEIRNGQIDVPGGILFGATISFSCNTGYKLFGSTSSFCLISGSSVQWSDPLPECREIYCPAPPQIDNGIIQGERDH YGYRQSVTYACNKGFTMIGEHSIYCTVNNDEGEWSGPPPECRG |
| SEQ ID NO:12 | EDCNELPPRRNTEILTGSWSOQTYPEGTQAIYKCRPGYRSLGNVIMVCRKGEWVALNPLRKCQKRP |
| SEQ ID NO:13 | EDCNELPPRRNTEILTGSWSDQTYPEGTQAIYKCRPGYRSLGNVIMVCRKGEWVALNPLRKCQK |
| SEQ ID NO:14 | EDCNELPPRRNTEILTGSWSDQTYPEGTQAIYKCRPGYRSLGNIIMVCRKGEWVALNPLRKCQKRP |
| SEQ ID NO:15 | EDCNELPPRRNTEILTGSWSDQTYPEGTQAIYKCRPGYRSLGNIIMVCRKGEWVALNPLRKCQK |
| SEQ ID NO:16 | GHHHHHH |
| SEQ ID NO:17 | MGWSCIILFLVATATGVHS |
| SEQ ID NO:18 | EDCNELPPRRNTEILTGSWSDQTYPEGTQAIYKCRPGYRSLGNVIMVCRKGEWVALNPLRKCQKRPCNPGEIRNG QIDVPGGILFGATISFSCNTGYKLFGSTSSFCLISGSSVQWSDPLPECREIYCPAPPQIDNGIIQGERDHYGYRQSVTYA CNKGFTMIGEHSIYCTVNNDEGEWSGPPPECRG |
| SEQ ID NO:19 | EDCNELPPRRNTEILTGSWSDQTYPEGTQAIYKCRPGYRSLGNVIMVCRKGEWVALNPLRKCQKKSCPNPGEIRNG QIDVPGGILFGATISFSCNTGYKLFGSTSSFCLISGSSVQWSDPLPECREIYCPAPPQIDNGIIQGERDHYGYRQSVTYA CNKGFTMIGEHSIYCTVNNDEGEWSGPPPECRG |
| SEQ ID NO:20 | EDCNELPPRRNTEILTGSWSDQTYPEGTQAIYKCRPGYRSLGNIIMVCRKGEWVALNPLRKCQKKSCPNPGEIRNG QIDVPGGILFGATISFSCNTGYKLFGSTSSFCLISGSSVQWSDPLPECREIYCPAPPQIDNGIIQGERDHYGYRQSVTYA CNKGFTMIGEHSIYCTVNNDEGEWSGPPPECRG |
| SEQ ID NO:21 | EDCNELPPRRNTEILTGSWSDQTYPEGTQAIYKCRPGYRSLGNIIMVCRKGEWVALNPLRKCQKRPCNPGEIRNG QIDVPGGILFGATISFSCNTGYKLFGSTSSFCLISGSSVQWSDPLPECREIYCPAPPQIDNGIIQGERDHYGYRQSVTYA CNKGFTMIGEHSIYCTVNNDEGEWSGPPPECRG |
| SEQ ID NO:22 | EDCNELPPRRNTEILTGSWSDQTYPEGTQAIYKCRPGYRSLGNVIMVCRKGEWVALNPLRKCQKRPCNPGEIRNG QIDVPGGILFGATISFSCNTGYKLFGSTSSFCLISGSSVQWSDPLPECREIYCPAPPQIDNGIIQGERDHYGYRQSVTYA CNKGFTMIGEHSIYCTVNNDEGEWSGPPPECRGGHHHHHH |
| SEQ ID NO:23 | EDCNELPPRRNTEILTGSWSDQTYPEGTQAIYKCRPGYRSLGNVIMVCRKGEWVALNPLRKCQKKSCPNPGEIRNG QIDVPGGILFGATISFSCNTGYKLFGSTSSFCLISGSSVQWSDPLPECREIYCPAPPQIDNGIIQGERDHYGYRQSVTYA CNKGFTMIGEHSIYCTVNNDEGEWSGPPPECRGGHHHHHH |
| SEQ ID NO:24 | EDCNELPPRRNTEILTGSWSDQTYPEGTQAIYKCRPGYRSLGNIIMVCRKGEWVALNPLRKCQKKSCPNPGEIRNG QIDVPGGILFGATISFSCNTGYKLFGSTSSFCLISGSSVQWSDPLPECREIYCPAPPQIDNGIIQGERDHYGYRQSVTYA CNKGFTMIGEHSIYCTVNNDEGEWSGPPPECRGGHHHHHH |
| SEQ ID NO:25 | EDCNELPPRRNTEILTGSWSDQTYPEGTQAIYKCRPGYRSLGNIIMVCRKGEWVALNPLRKCQKRPCNPGEIRNG QIDVPGGILFGATISFSCNTGYKLFGSTSSFCLISGSSVQWSDPLPECREIYCPAPPQIDNGIIQGERDHYGYRQSVTYA CNKGFTMIGEHSIYCTVNNDEGEWSGPPPECRGGHHHHHH |
| SEQ ID NO:26 | MGWSCIILFLVATATGVHSEDCNELPPRRNTEILTGSWSDQTYPEGTQAIYKCRPGYRSLGNVIMVCRKGEWVALNP LRKCQKRPCPNPGEIRNGQIDVPGGILFGATISFSCNTGYKLFGSTSSFCLISGSSVQWSDPLPECREIYCPAPPQIDN GIIQGERDHYGYRQSVTYACNKGFTMIGEHSIYCTVNNDEGEWSGPPPECRGGHHHHHH |
| SEQ ID NO:27 | MGWSCIILFLVATATGVHSEDCNELPPRRNTEILTGSWSDQTYPEGTQAIYKCRPGYRSLGNVIMVCRKGEWVALNP LRKCQKKSCPNPGEIRNGQIDVPGGILFGATISFSCNTGYKLFGSTSSFCLISGSSVQWSDPLPECREIYCPAPPQIDN GIIQGERDHYGYRQSVTYACNKGFTMIGEHSIYCTVNNDEGEWSGPPPECRGGHHHHHH |
| SEQ ID NO:28 | MGWSCIILFLVATATGVHSEDCNELPPRRNTEILTGSWSDQTYPEGTQAIYKCRPGYRSLGNIIMVCRKGEWVALNP LRKCQKKSCPNPGEIRNGQIDVPGGILFGATISFSCNTGYKLFGSTSSFCLISGSSVQWSDPLPECREIYCPAPPQIDN GIIQGERDHYGYRQSVTYACNKGFTMIGEHSIYCTVNNDEGEWSGPPPECRGGHHHHHH |
| SEQ ID NO:29 | MGWSCIILFLVATATGVHSEDCNELPPRRNTEILTGSWSDQTYPEGTQAIYKCRPGYRSLGNIIMVCRKGEWVALNP LRKCQKRPCNPGEIRNGQIDVPGGILFGATISFSCNTGYKLFGSTSSFCLISGSSVQWSDPLPECREIYCPAPPQIDN GIIQGERDHYGYRQSVTYACNKGFTMIGEHSIYCTVNNDEGEWSGPPPECRGGHHHHHH |
| SEQ ID NO:30 | MGWSCIILFLVATATGVHSEDCNELPPRRNTEILTGSWSDQTYPEGTQAIYKCRPGYRSLGNVIMVCRKGEWVALNP LRKCQKRPCPNPGEIRNGQIDVPGGILFGATISFSCNTGYKLFGSTSSFCLISGSSVQWSDPLPECREIYCPAPPQIDN GIIQGERDHYGYRQSVTYACNKGFTMIGEHSIYCTVNNDEGEWSGPPPECRG |
| SEQ ID NO:31 | MGWSCIILFLVATATGVHSEDCNELPPRRNTEILTGSWSDQTYPEGTQAIYKCRPGYRSLGNVIMVCRKGEWVALNP LRKCQKKSCPNPGEIRNGQIDVPGGILFGATISFSCNTGYKLFGSTSSFCLISGSSVQWSDPLPECREIYCPAPPQIDN GIIQGERDHYGYRQSVTYACNKGFTMIGEHSIYCTVNNDEGEWSGPPPECRG |
| SEQ ID NO:32 | MGWSCIILFLVATATGVHSEDCNELPPRRNTEILTGSWSDQTYPEGTQAIYKCRPGYRSLGNIIMVCRKGEWVALNP LRKCQKKSCPNPGEIRNGQIDVPGGILFGATISFSCNTGYKLFGSTSSFCLISGSSVQWSDPLPECREIYCPAPPQIDN GIIQGERDHYGYRQSVTYACNKGFTMIGEHSIYCTVNNDEGEWSGPPPECRG |
| SEQ ID NO:33 | MGWSCIILFLVATATGVHSEDCNELPPRRNTEILTGSWSDQTYPEGTQAIYKCRPGYRSLGNIIMVCRKGEWVALNP LRKCQKRPCNPGEIRNGQIDVPGGILFGATISFSCNTGYKLFGSTSSFCLISGSSVQWSDPLPECREIYCPAPPQIDN GIIQGERDHYGYRQSVTYACNKGFTMIGEHSIYCTVNNDEGEWSGPPPECRG |
| SEQ ID NO:34 | QCNAPEWLPFARPTNLTDEFEFPIGTYLKYECRPGYYGRPFSIICLKNSVWTGAKDRCRRKSCRNPPDPVNGMVHV IKDIQFGSQIKYSCTKGYRLIGSSSATCIISGNTVIWDNETPICDRIPCGLPPTITNGDFISTNRENFHYGSVVTYRCNPG SGGRKVFELVGEPSIYCTSNDDQVGIWSGPAPQCIGHHHHHH |
| SEQ ID NO:35 | SCEVPTRLNSASLKQPYITQNYFPVGTVVEYECRPGYRREPSLSPKLTCLQNLKWSTAVEFCKKKSCPNPGEIRNGQI DVPGGILFGATISFSCNTGYKLFGSTSSFCLISGSSVQWSDPLPECREIYCPAPPQIDNGIIQGERDHYGYRQSVTYAC NKGFTMIGEHSIYCTVNNDEGEWSGPPPECRGGHHHHHH |
| SEQ ID NO:36 | EDCNELPPRRNTEILTGSWSDQTYPEGTQAIYKCRPGYRSLGNIIMVCRKGEWVALNPLRKCQKRPCGHPGDTPFG TFTLTGGNVFEYGVKAVYTCNEGYQLLGEINYRECDTDGWTNDIPICEVVKCLPVTAPENGKIVSSAMEPDREYHFG QAVRFVCNSGYKIEGDEEMHCSDDGFWSKEKPKCVEISCKSPDVINGSPISQKIIYKENERFQYKCNMGYEYSERGD AVCTESGWRPLPSCEEKSCDNPYIPNGDYSPLRIKHRTGDEITYQCRNGFYPATRGNTAKCTSTGWIPAPRCTLKPG HHHHHH |
| SEQ ID NO:37 | GHCQAPDHFLFAKLKTQTNASDFPIGTSLKYECRPEYYGRPFSITCLDNLVWSSPKDVCKRKSCPNPGEIRNGQIDVP GGILFGATISFSCNTGYKLFGSTSSFCLISGSSVQWSDPLPECREIYCPAPPQIDNGIIQGERDHYGYRQSVTYACNKGF TMIGEHSIYCTVNNDEGEWSGPPPECRGGHHHHHH |
| SEQ ID NO:38 | EDCNELPPRRNTEILTGSWSDQTYPEGTQAIYKCRPGYRSLGNIIMVCRKGEWVALNPLRKCQKRPCGHPGDTPFG TFTLTGGNVFEYGVKAVYTCNEGYQLLGEINYRECDTDGWTNDIPICEVVKCLPVTAPENGKIVSSAMEPDREYHFG QAVRFVCNSGYKIEGDEEMHCSDDGFWSKEKPKCVEISGHHHHHH |
| SEQ ID NO:39 | DCGLPPDVPNAQPALEGRTSFPEDTVITYKCEESFVKIPGEKDSVICLKGSQWSDIEEFCNRSCEVPTRLNSASLKQPY ITQNYFPVGTVVEYECRPGYRREPSLSPKLTCLQNLKWSTAVEFCKKKSCPNPGEIRNGQIDVPGGILFGATISFSCNT GYKLFGSTSSFCLISGSSVQWSDPLPECREIYCPAPPQIDNGIIQGERDHYGYRQSVTYACNKGFTMIGEHSIYCTVN NDEGEWSGPPPECRGKSGHHHHHH |

## Claims

1. A hybrid protein, which comprises or consists of
(i) CCP1 of human complement Factor H (CFH),
(ii) CCP3 and CCP4 of human decay-accelerating factors (DAFs), and
optionally a signal peptide and/or a tag.

2. The hybrid protein of claim 1, wherein the CCP3 and CCP4 of DAF are directly linked together to form CCP3-4.

3. The hybrid protein of claim 1 or 2, wherein the CCP1 of human CFH comprises or consists of the amino acid sequence at positions 19-82 of the human CFH protein, optionally, the CCP1 comprises a V62I mutation, wherein the amino acid positions are numbered corresponding to the amino acid positions as set forth in SEQ ID NO: 3.

4. The hybrid protein of claim 1 or 2, wherein the CCP1 of human CFH comprises or consists of the amino acid sequence at positions 19-84 of the human CFH protein, optionally, the CCP1 comprises a V62I mutation, wherein the amino acid positions are numbered corresponding to the amino acid positions as set forth in SEQ ID NO: 3.

5. The hybrid protein of any one of claims 1-4, wherein the CCP3 of human DAF comprises or consists of the amino acid sequence at positions 161-222 of the human DAF protein, and/or the CCP4 of human DAF comprises or consists of the amino acid sequence at positions 223-285 of the human DAF protein, wherein the amino acid positions are numbered corresponding to the amino acid positions as set forth in SEQ ID NO: 1.

6. The hybrid protein of any one of claims 1-4, wherein the CCP3 of human DAF comprises or consists of the amino acid sequence 163-222 of the human DAF protein, and/or the CCP4 of human DAF comprises or consists of the amino acid sequence 223-285 of the human DAF protein, wherein the amino acid positions are numbered corresponding to the amino acid positions as set forth in SEQ ID NO: 1.

7. The hybrid protein of claim 2, wherein the CCP3-4 of human DAF comprises or consists of the amino acid sequence at positions 161-285 of the human DAF protein, wherein the amino acid positions are numbered corresponding to the amino acid positions as set forth in SEQ ID NO: 1.

8. The hybrid protein of any one of claims 2-4, wherein the CCP3-4 of human DAF comprises or consists of the amino acid sequence at position 163-285 of the human DAF protein, wherein the amino acid positions are numbered corresponding to the amino acid positions as set forth in SEQ ID NO: 1.

9. The hybrid protein of claim 2, wherein
(1) the CCP1 of human CFH comprises or consists of the amino acid sequences at positions 19-82 of the human CFH protein, and the CCP3 of human DAF comprises or consists of the amino acid sequence 161-222 of the human DAF protein, and the CCP4 of human DAF comprises or consists of the amino acid sequence 223-285 of the human DAF protein;
(2) the CCP1 of human CFH comprises or consists of the amino acid sequence at positions 19-84 of the human CFH protein, and the CCP3 of human DAF comprises or consists of the amino acid sequence 163-222 of the human DAF protein, and the CCP4 of human DAF comprises or consists of the amino acid sequence 223-285 of the human DAF protein;
(3) the CCP1 of human CFH comprises or consists of the amino acid sequence at positions 19-82 of the human CFH protein, and the CCP3-4 of human DAF comprises or consists of the amino acid sequence at positions 161-285 of the human DAF protein; or
(4) the CCP1 of human CFH comprises or consists of the amino acid sequence at positions 19-84 of the human CFH protein, and the CCP3-4 of human DAF comprises or consists of the amino acid sequence at positions 163-285 of the human DAF protein;
wherein the amino acid positions of the human CFH protein are numbered corresponding to the amino acid positions as set forth in SEQ ID NO: 3 and the amino acid positions of the human DAF protein are numbered corresponding to the amino acid positions as set forth in SEQ ID NO: 1.

10. The hybrid protein of claim 9, wherein the CCP1 of human CFH has a V62I mutation.

11. The hybrid protein of any one of claims 1-10, wherein the human CFH protein is a native human CFH protein or comprises
(i) the amino acid sequence as set forth in SEQ ID NO: 3 or 5;
(ii) the amino acid sequence encoded by the nucleotide sequence as set forth in SEQ ID NO: 4 or 6;
(iii) the amino acid sequence having at least 95%, 96%, 97%, 98%, or 99% identity to the amino acid sequence as set forth in (i) or (ii); or
consists of the amino acid sequence as set forth in any one of (i) to (iii).

12. The hybrid protein of any one of claims 1-11, wherein the human DAF protein is a native human DAF protein or comprises
(i) the amino acid sequence as set forth in SEQ ID NO: 1;
(ii) the amino acid sequence encoded by the nucleotide sequence as set forth in SEQ ID NO: 2;
(iii) the amino acid sequence having at least 95%, 96%, 97%, 98%, or 99% identity to the amino acid sequence as set forth in (i) or (ii); or
(iv) consists of the amino acid sequence as set forth in any one of (i) to (iii).

13. The hybrid protein of any one of claims 1-12, wherein
the CCP1 of human CFH comprises or consists of the amino acid sequence as set forth in SEQ ID NO: 12, 13, 14, or 15, or an amino acid sequence having at least 95%, 96%, 97%, 98%, or 99% identity to the amino acid sequence as set forth in SEQ ID NO: 12, 13, 14, or 15;
the CCP3 of human DAF comprises or consists of the amino acid sequence as set forth in SEQ ID NO: 7 or 8, or an amino acid sequence having at least 95%, 96%, 97%, 98%, or 99% identity to the amino acid sequence as set forth in SEQ ID NO: 7 or SEQ ID NO: 8;
the CCP4 of human DAF comprises or consists of the amino acid sequence as set forth in SEQ ID NO: 9, or an amino acid sequence having at least 95%, 96%, 97%, 98%, or 99% identity to the amino acid sequence as set forth in SEQ ID NO: 9; and/or
the CCP3-4 of human DAF comprises or consists of the amino acid sequence as set forth in SEQ ID NO: 10 or SEQ ID NO: 11, or an amino acid sequence having at least 95%, 96%, 97%, 98%, or 99% identity to the amino acid sequence as set forth in SEQ ID NO: 10 or SEQ ID NO: 11.

14. The hybrid protein of any one of claims 1-13, wherein the tag is, for example, a purification tag such as a hexahistidine tag or a biotin tag, comprising, e.g., the amino acid sequence as set forth in SEQ ID NO: 12.

15. The hybrid protein of any one of claims 1-14, wherein the signal peptide is secretory, comprising, e.g., the amino acid sequence as set forth in SEQ ID NO: 17.

16. The hybrid protein of any one of claims 1-15, wherein the hybrid protein comprises or consists of an amino acid sequence of any one of SEQ ID NOs: 18-33, or an amino acid sequence having at least a 95%, 96%, 97%, 98%, or 99% identity to the amino acid sequence.

17. A nucleic acid molecule encoding the hybrid protein of any one of claims 1-16.

18. An expression vector comprising the nucleic acid molecule of claim 17, preferably the expression vector is pcDNA3.1.

19. A host cell comprising the nucleic acid molecule of claim 17 or the expression vector of claim 18, preferably the host cell is prokaryotic or eukaryotic, such as a 293 cell, e.g., an Expi293 cell.

20. A method of preparing the hybrid protein of any one of claims 1-16, the method comprising culturing the host cell of claim 19 under conditions suitable for expression of the hybrid protein, and optionally further comprising isolating the protein from the host cell or the host cell culture medium, and/or purifying the protein.

21. A pharmaceutical composition or medicament or formulation comprising the hybrid protein according to any one of claims 1-16 or a nucleic acid molecule encoding the same, or the nucleic acid molecule of claim 17, and optionally a pharmaceutically acceptable excipient.

22. A pharmaceutical combination product comprising
the hybrid protein according to any one of claims 1-16 or a nucleic acid molecule encoding the same, or the nucleic acid molecule of claim 17; and the additional therapeutic agents.

23. A method of preventing or treating a complement system-associated disease or disorder in a subject, comprising administering to the subject an effective amount of the hybrid protein of any one of claims 1-16 or a nucleic acid molecule encoding the same; or the nucleic acid molecule of claim 17; or the pharmaceutical composition or formulation of claim 21; or the pharmaceutical combination product of claim 22.

24. The method of claim 23, wherein the disease or disorder is due to an abnormal activation of the complement system or a dysregulation of the complement system.

25. The method of claim 24, wherein the abnormal activation of the complement system or the dysregulation of the complement system is due to, for example, a microbial infection or an increase in autoimmune antibodies, or a decrease, deletion, incapacitation, or interference or blockade of a function of a complement regulatory protein.

26. The method of claim 23, wherein the complement system-associated disease or disorder is selected from a disease requiring hemolysis inhibition, such as a disease requiring inhibition of hemolysis of the classical pathway of the complement immunity and/or the alternative pathway of the complement immunity; or a disease requiring inhibition of C3b deposition activity, such as dense deposition disease DDD.
